# EUROPEAN PATENT SPECIFICATION

(11) **EP 0 157 260 B1**
(45) Date of publication and mention of the grant of the patent: **13.06.1990**
(21) Application number: 85103017.1
(22) Date of filing: 15.03.1985
(51) Int. Cl.: C07D 471/04, A61K 31/435

(54) **4,7-Dihydropyrazolo(3,4-b)Pyridine derivatives**
4,7-Dihydropyrazolo[3,4-b]pyridin-Derivate
Dérivés de 4,7-dihydropyrazolo[3,4-b]pyridine

(30) Priority: 19.03.1984 JP 53118/84
(43) Date of publication of application: 09.10.1985
(73) Proprietor: SHIONOGI & CO., LTD., Osaka 541 (JP)
(72) Inventor: Adachi, Ikuo, Suita-shi Osaka (JP); Yamamori, Teruo, Takarazuku-shi Hyogo (JP); Ueda, Motohiko, Suita-shi Osaka (JP)
(74) Representative: Bruin, Cornelis Willem

(56) References cited:
- EP-A- 0 107 619
- EP-A- 0 114 273
- CH-A- 635 840
- DE-A- 1 695 928
- US-A- 3 485 847
- US-A- 3 985 757
- US-A- 4 038 283

## Description

This invention relates to pyrazolodihydropyridine derivatives which have potent anti hypertensive and coronary vasodilating activities based upon a calcium-blocking effect and which can be used in the treatment of cardiovascular diseases such as angina pectoris, hypertension, cerebrovascular dysfunctions or arrhytmia. Further, the invention relates to a method of producing such derivatives and to the utilisation thereof in pharmaceutical compositions.

It is well-known that many 1,4-dihydropyridine derivatives have a calcium-blocking effect leading to antihypertensive and coronary vasodilating activities and that they can be used with high efficiency in the treatment of cardiovascular diseases. Examples of such useful calcium-blockers are: Nifedipine (US-A-3,485,847 and US-A-3,644,627), Nisoldipine (JP-B-56-47185), 2-amino-1,4-dihydropyridine derivatives (JP-B-57-20306), Nicardipine (JP―A―49―109384), 2-pyridyl-1,4-dihydropyridine derivatives (JP-A-54-48796), 2-methyldihydropyridine derivatives (JP-A-55-62055).

In accordance with the invention, it has now been found that a group of novel pyrazolodihydropyridine derivatives also shows excellent anti hypertensive and coronary vasodilating activities based upon a calcium blocking effect and that such activities are not accompanied by a systole inhibitory action therein. This means that the newly found compounds do not have the disadvantage of the conventional calcium-blockers and that they can be used with more advantage in the treatment of cardiovascular diseases.

The pyrazolodihydropyridine derivatives of the invention can be represented by the formula (I) 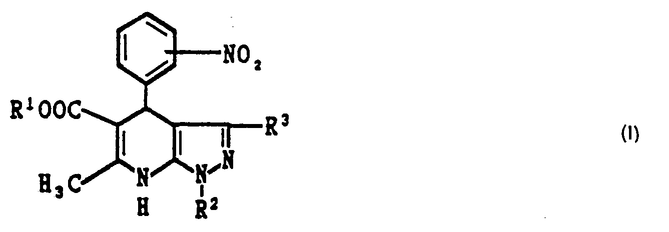 wherein R' is (1) C₇―C₈ alkyl, (2) C₄―C₆ cycloalkyl which may be substituted by C₁―C₅ alkyls, (3) C₃―C₇ cycloa)kyl(C₁―C₄)aikyt, (4) C₁―C₄ alkoxy(C₁―C₄)alkyl, (5) C₄―C₇ cycloalkyloxy(C₁―C₄)alkyl, (6) phenoxy(C₁―C₄)alkyl, (7) C₁―C₄ alkylthio(C₁―C4₎alkyl, (8) C₄―C₇ cycloalkylthio(C₁―C₄alkyl, (9) phenylthio(C₁―C₄)alkyl, (10) mono- or di-(C₁―C₄)alkylamino (C₁―C₄)alkyl, (11) tetrahydrofuryl-(C₁―C₄)alkyl, (12) phenyl(C₁―C₃)alkyl which may be substituted by one or two substituents selected from halogens and Cₗ-C₄ alkoxy, (13) N-benzylpyrrolidinyl, or (14) N-benzylpiperidinyl; R² is Cₗ-C₄ alkyl; and R³ is C₄―C₆ cycloalkyl or C₃―C₇ cycloalkyl(C₁―C₄)alkyl; and acid-addition salts thereof.

It should be noted that other groups of pyrazolodihydropyridine derivatives having similar activities (in one case including the absence of systole inhibitory activity) were disclosed already in EP-A-0107619 and EP-A-0114273, both published after the priority date of the present invention.

The derivatives (I) of the present invention can readily be produced by the reaction of an α,β-unsaturated ketone reagent (II) with a 5-aminopyrazole (III), as shown in the following scheme: 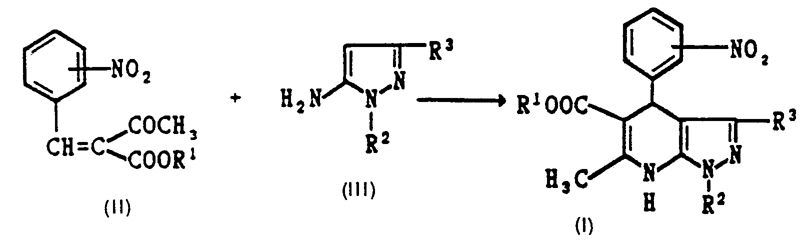 wherein R', R² and R³ have the same meanings as defined above. This is in fact a Michael addition accompanied by cyclisation.

The reaction may be accomplished in the absence or presence of any solvent. Suitable solvents for this reaction are alcohols such as methanol, ethanol, isopropanol, tert-butanol or ethylene glycol; hydrocarbons such as benzene, toluene, or xylene; ethers such as ether, tetrahydrofuran, dioxane, glyme or diglyme; halogeno hydrocarbons such as dichloromethane, chloroform, dichloroethane or carbon tetrachloride; esters such as ethyl acetate; acetic acid; dimethylformamide; or pyridine. An acid or an organic base is employed as a catalyst, if necessary. Suitable acids are inorganic acids such as e.g., sulfuric acid, hydrochloric acid, phosphoric acid and the like; para-toluenesulfonic acid, acetic acid, or formic acid as an organic acid; and boron trifluoride, zinc chloride, aluminium chloride, magnesium chloride, or tin chloride as a Lewis acid. Suitable bases are organic bases such as e.g., triethylamine, pyridine, pyrrolidine, or piperidine.

The reaction is completed after a few hours or a few days at room temperature (1-30°C) or under heating (30-100°C).

The starting compounds for the reaction, namely the 5-aminopyrazole and the α,β-unsaturated ketone reagent, may be prepared in the following way.

### (i) Preparation of 5-aminopyrazole (III):

A 5-aminopyrazole (III) can be produced according to the reaction sequence shown below. In other words, it can be prepared in a high yield by cyclization of a hydrazine (IV) with a β-ketonitrile (V).

Processes for the production of the compounds (III) are disclosed in EP-A-0 107 619.

The β-ketonitriles (V) are prepared through the reaction of an acid chloride or an ester, with an alkali metal salt of acetonitrile. 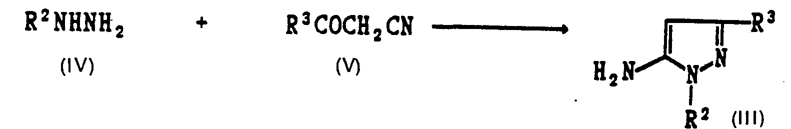 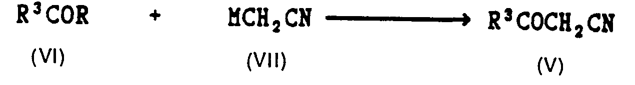 In this reaction sequence R² and R³ have the same meanings as defined above, R represents a halogen or an ester residue, and M represents an alkali metal.

### (ii) Preparation of α,β-unsaturated ketone reagents (II):

α,β-Unsaturated ketone reagents (II) are prepared, as shown in the following scheme, by condensation of an aldehyde (VIII) with an acetoacetic ester (IX); this type of reaction is disclosed in J. Chem. Soc., 81, 1212 (1902); Chem. Ber., 29, 172 (1896); Ann., 218, 170 (1883); J. Chem. Soc., 3092 (1962). 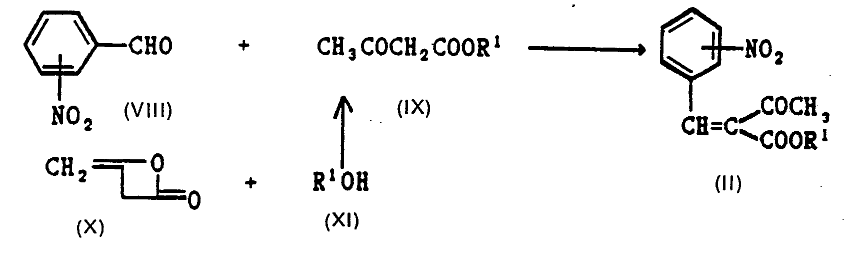 In this scheme R' has the same meaning as defined above.

The acetoacetic esters are prepared by reaction of a diketone (X) with an alcohol (XI) in the presence of a catalyst in the form of an acid# (hydrochloric acid, sulfuric acid, phosphoric acid and the like) or a base (pyridine, pyrrolidine, piperidine and the like).

Examples of the compounds of the present invention, which can be prepared by reaction of the above- obtained α,β-unsaturated ketone reagents (II) with a 5-aminopyrazole (III), are listed below.
(1) 2-Methoxyethyl3-cyclopentyl-1,6-dimethyl-4-(2-nitrophenyl)-4,7-dihydropyrazolo[3,4-b]pyridine-5-carboxylate,
(2) 2-Methoxyethyl 3-cyclopentyl-1,6-dimethyl-4-(3-nitrophenyl)-4,7-dihydropyrazolo[3,4-b]pyridine-5-carboxylate,
(3) 2-Methoxyethyl 3-cyclohexyl-1,6-dimethyl-4-(2-nitrophenyl)-4,7-dihydropyrazolo(3,4-b]pyridine-5-carboxylate,
(4) 2-Methoxyethyl 3-cyclopentylmethyl-1,6-dimethyl-4-(2-nitrophenyl)-4,7-dihydropyrazolo[3,4-b]pyridine-5-carboxylate,
(5) 2-Isopropoxypropyl 3-cyclopentyl-1,6-dimethyl-4-(2-nitrophenyl)-4,7-dihydropyrazolo[3,4-b]pyridine-5-carboxylate,
(6) 2-Cyclopentyloxyethyl 3-cyclopentyl-1,6-dimethyl-4-(2-nitrophenyl)-4,7-dihydropyrazolo[3,4-b]pyridine-5-carboxylate,
(7) 2-Cyclohexyloxyethyl 3-cyclopentyl-1,6-dimethyl-4-(2-nitrophenyl)-4,7-dihydropyrazolo(3,4-b]pyridine-5-carboxylate,
(8) 2-Tetrahydrofurylmethyl 3-cyclopentyl-1,6-dimethyl-4-(2-nitrophenyl)-4,7-dihydropyrazolo[3,4-b]pyridine-5-carboxylate,
(9) 2-Phenoxyethyl 3-cyclopentyl-1,6-dimethyl-4-(2-nitrophenyl)-4,7-dihydropyrazolo[3,4-b]pyridine-5-carboxylate,
(10) 2-Phenoxyethyl 3-cyclopentyl-1,6-dimethyl-4-(3-nitrophenyl)-4,7-dihydropyrazolo[3,4-b]pyridine-5-carboxylate,
(11) 2-Phenoxyethyl 3-cyclohexyl-1,6-dimethyl-4-(2-nitrophenyl)-4,7-dihydropyrazolo[3,4-b]pyridine-5-carboxylate,
(12) 2-Methylthioethyl 3-cyclopentyl-1,6-dimethyl-4-(2-nitrophenyl)-4,7-dihydropyrazolo[3,4-b]pyridine-5-carboxylate,
(13) 3-dimethylaminopropyl 3-cyclopentyl-1,6-dimethyl-4-(2-nitrophenyl)-4,7-dihydropyrazolo[3,4-b]pyridine-5-carboxylate,
(14) N-Benzylpyrrolidin-3-yl3-cyclopentyl-1,6-dimethyl-4-(2-nitrophenyl)-4,7-dihydropyrazolo[3,4-b]pyridine-5-carboxylate,
(15) N-Benrylpiperidin-4-yl 3-cyclopentyl-1,6-dimethyl-4-(2-nitrophenyl)-4,7-dihydropyrazolo[3,4-b]pyridine-5-carboxylate,
(16) Phenethyl 3-cyclopentyl-1,6-dimethyl-4-(2-nitrophenyl)-4,7-dihydropyrazolo[3,4-b]pyridine-5-carboxylate,
(17) 4-Chlorophenethyl 3-cyclopentyl-1,6-dimethyl-4-(2-nitrophenyl)-4,7-dihydropyrazolo[3,4-b]pyridine-5-carboxylate,
(18) 3,4-Dimethoxyphenethyl 3-cyclopentyl-1,6-dimethyl-4-(2-nitrophenyl)-4,7-dihydropyrazolo(3,4-b]pyridine-5-carboxylate,
(19) 1-Methoxy-2-phenylethyl 3-cyclopentyl-1,6-dimethyl-4-(3-nitrophenyl)-4,7-dihydropyrazolo[3,4-b]pyridine-5-carboxylate,
(20) 2-Isopropyl-4-methylcyclohexyl 3-cyclopentyl-1,6-dimethyl-4-(3-nitrophenyl)-4,7-dihydropyrazolo[3,4-b]pyridine-5-carboxylate,
(21) Phenylthioethyl3-cyclopentyl-1,6-dimethyl-4-(2-nitrophenyl)-4,7-dihydropyrazolo[3,4-b]pyridine-5-carboxylate,
(22) 2-Isopropylthioethyl 3-cyclopentyl-1,6-dimethyl-4-f2-nitrophenyl)-4,7-dihydropyrazolo[3,4-b]pyridine-5-carboxylate,
(23) 2-Cyclopentylthioethyl 3-cyclopentyl-1,6-dimethyl-4-(2-nitrophenyl)-4,7-dihydropyrazolo[3,4-b]pyridine-5-carboxylate,
(24) 2-Cyclopentylethyl 3-cyclopentyl-1,6-dimethyl-4-(2-nitrophenyl)-4,7-dihydropyrazolo[3,4-b]pyridine-5-carboxylate,
(25) Cyclohexylmethyl 3-cyclopentyl-1,6-dimethyl-4-(2-nitrophenyl)-4,7-dihydropyrazolo[3,4-b]pyridine-5-carboxylate,
(26) Cyclopentyl 3-cyclopentyl-1,6-dimethyl-4-(2-nitrophenyl)-4,7-dihydropyrazolo[3,4-b]pyridine-5-carboxylate,
(27) Cyclohexyl 3-cyclopentyl-1,6-dimethyl-4-(2-nitrophenyl)-4,7-dihydropyrazolo[3,4-b]pyridine-5-carboxylate.

In addition thereto, the acid addition salts derived from the above-listed compounds, are part of this invention. Some examples of the acids capable to form such salts are inorganic acids such as hydrohalogenic acid (hydrochloric acid, hydrobromic acid or the like), sulfuric acid, nitric acid or phosphoric acid and organic acids such as acetic acid, propionic acid, oxalic acid, malonic acid, succinic acid, glutaric acid, adipic acid, malic acid, maleic acid, fumaric acid, citric acid, benzoic acid or methanesulfonic acid.

The compounds of the present invention have excellent antihypertensive and coronary vasodilating actions derived from a Ca-blocking effect, but have no systole inhibitory action which has been a defect of the conventional Ca-blockers. This can be illustrated by the following biological tests.

### (Compounds Tested)

(A) Nifedipine
(B): 2-)Methoxyethy)3-cyclopentyl-1,6-dimethyl-4-(2-nitrophenyl)-4,7-dihydropyrazolo[3,4-b]pyridine-5-carboxylate
(C): 2-Methoxyethyl 3-cyclopentyl-1,6-dimethyl-4-(3-nitrophenyl)-4,7-dihydropyrazolo[3,4-b]pyridine-5-carboxylate
(D): 2-Methoxyethyl 3-cyclohexyl-1,6-dimethyl-4-(2-nitrophenyl)-4,7-dihydropyrazolo(3,4-b]pyridine-5-carboxylate
(E): 2-Methoxyethyl 3-cyclopentylmethyl-1,6-dimethyl-4-(2-nitrophenyl)-4,7-dihydropyrazolo[3,4-b]pyridine-5-carboxylate
(F): 3-lsopropoxypropyl 3-cyclopentyl-1,6-dimethyl-4-(2-nitrophenyl)-4,7-dihydropyrazolo[3,4-b]pyridine-5-carboxylate
(G): 2-Cyclopentyloxyethyl 3-cyclopentyl-1,6-dimethyl-4-(2-nitrophenyl)-4,7-dihydropyrazolo[3,4-b]pyridine-5-carboxylate
(H): 2-Tetrahydrofurylmethyl 3-cyclopentyl-1,6-dimethyl-4-(2-nitrophenyl)-4,7-dihydropyrazolo[3,4-b]pyridine-5-carboxylate
(I): 2-Phenoxyethyl 3-cyclopentyl-1,6-dimethyl-4-(2-nitrophenyl)-4,7-dihydropyrazolo[3,4-b]pyridine-5-carboxylate
(J): 2-Methylthioethyl 3-cyclopentyl-1,6-dimethyl-4-(2-nitrophenyl)-4,7-dihydropyrazolo[3,4-b]pyridine-5-carboxylate
(K): Phenethyl 3-cyclopentyl-1,6-dimethyl-4-(2-nitrophenyl)-4,7-dihydropyrazolo[3,4-b]pyridine-5-carboxylate
(L): 4-Chlorophenethyl 3-cyclopentyl-1,6-dimethyl-4-(3-nitrophenyl)-4,7-dihydropyrazolo[3,4-b]pyridine-5-carboxylate
(M): 3,4-Dimethoxyphenethyl 3-cyclopentyl-1,6-dimethyl-4-(3-nitrophenyl)-4,7-dihydropyrazolo[3,4-b]pyridine-5-carboxylate

### Test Method)

### (1) Antihypertensive Action:

Female Spontaneously Hypertensive Rats (SH rats) having a systolic blood pressure of 160 mm Hg were employed without anaesthetization. The systolic pressure was bloodlessly measured by the tail-cuff method (Japan J. Pharmacol., 28, 617 (1978)) using a Physiograph and an Electrosphygmomanometer (DMP-4B, PE-300, Narco Biosystems, Inc., Houston), after keeping the rats warm at 50°C for 2-3 minutes. Each compound was intraperitoneally administered to the rats at a dose of 3 mg per kilogram.

### (2) Coronary Vasodilating Action and Systole Inhibitory Action:

Guinea pigs of 400-800 g body weight were strongly hit on their heads and the cartoid artery of each Guinea pig was cut down in order to make it bloodless. The heart was isolated and perfused with liquid at a constant pressure (50 cm H₂0) by the Langendorff method [Basic Pharmacology & Therapeutics, 9(4), 181 (1981)]. A Krebs-Ringer bicarbonate solution (27°C) containing 0.5% defibrinated blood was employed as a perfusate, into which a mixed gas of 95% oxygen and 5% carbon dioxide was continuously introduced. The flowing perfusate was led into a drop counter, and the changes of the flow i.e. increase and decrease were taken as indications for coronary vasodilation and vasoconstriction, respectively. The isomeric contraction of the apex and the number of drops of the coronary perfusate were recorded on a Recticorder (RJG 3006, Nihon Koden) by way of an F-D Pick-up (SB-1T, Nihon Koden). Each of the test compounds at a dose of 0.1 µg was administered through a short rubber tube connecting the aorta and the cannula.

Results:
An antihypertensive action is shown in a maximum decrease of blood pressure, i.e., a maximum difference between systolic pressures after and before the administration of the test compound; along with which the duration period is also shown.

A coronary vasodilating action is shown in changes of the quantity of the perfusate, and a systole inhibitory action is shown in changes of the inotropic tension.

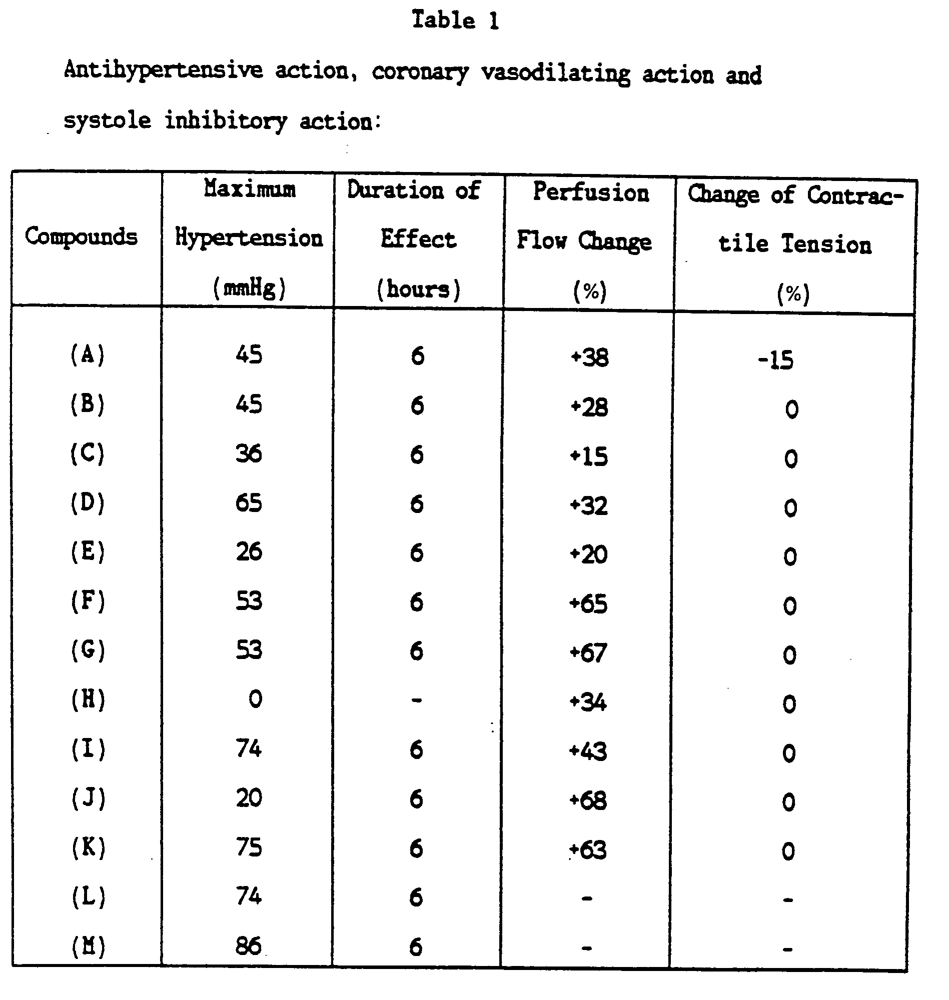

Since the compounds of this invention, as clearly seen from the above-listed results, have strong antihypertensive and coronary vasodilating actions but no systole inhibitory action, they can be used as cardiovascular agents with less adverse reactions to human or animals.

_{.} The compounds of this invention and the acid addition salts thereof can orally or parenterally be administered to human or animals and can be manufactured in various formulations in compliance with the usage. Thus, for instance, they can be formulated to tablets, capsules, pills, granules, fine granules, aqueous solutions, emulsions or the like. In the course of the formulation, conventional carriers or diluents such as lactose, sucrose, starch, cellulose, talc, magnesium stearate, magnesium oxide, calcium sulfate, powdered gum arabic, agelatin, sodium alginate, sodium benzoate, stearic acid and the like are employed. Injection compositions may be formulated as a solution in distilled water, saline solution, Ringer solution or the like, or a suspension in sesame oil.

The compounds of this invention may be administered to an adult orally at a dose of 1-50 mg a day, or intravenously at 0.5-20 mg a day.

The present invention is further described in the following Examples and Preparations.

### Example 1

Preparations of 2-methoxyethyl 3-cyclopentyl-1,6-dimethyl-4-(2-nitrophenyl)-4,7-dihydropyrazolo[3,4-b]pyridine-5-carboxylate 3.

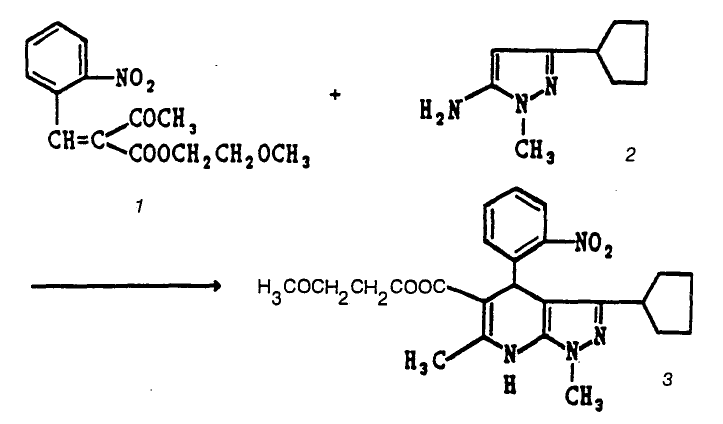

In 6 ml of tert-butanol are dissolved 0.90 g (3.05 mMol.) of 2-methoxyethyl 2-nitrobenzylideneacetoacetate 1 and 0.50 g (3.05 mMol.) of 3-cyclopentyl-5-amino-1-methylpyrazole 2. The solution is allowed to react at 80°C for 3 hours, and then evaporated under reduced pressure. The resulting residue is crystallized from ether, filtered and washed with a small amount of ether to give 1.15 g of the title compound 3. This is recrystallized from ethanol to give 0.92 g of yellow prisms in 59.6% yield, mp. 196-198°C.

Elementary Analysis: Calcd. (%) for C₂₃H₂₈N₄O₅: C, 62.71; H, 6.41; N, 12.72. Found (%): C, 62.69; H, 6.20; N, 12.82.
IR(Nujol) vmax: 3260(NH), 1690(C=O), 1360(N0₂) cm⁻¹.
NMR(CDCl₃) 5ppm: 0.97-2.03 (8H, m), 2.33,3.27,3.63 (3H x 3, s), 2.92 (1H, m), 3.51 (2H, m), 4.13 (2H, m), 5.92 (1H, s), 6.67 (1H, br, s), 7.00-7.73 (4H, m).

### Examples 2-32

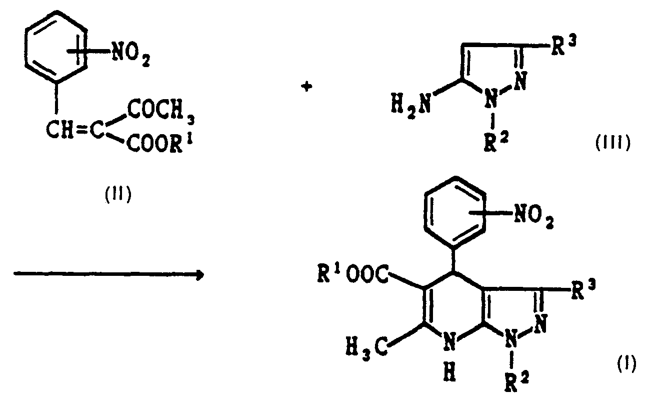

wherein R¹, R² and R³ each have the same meaning as defined above.

A solution of compounds (II) and (III) in a solvent is allowed to react in nitrogen atmosphere at room temperature or under heating and then evaporated. The residue is crystallized from ether, or chromatographed on silica gel to give the objective compound (I). This, if needed, is purified by recrystallization.

The objective compounds of the present invention which are listed in the following Table 2, can be prepared in the manner commonly described above. Details of the reaction conditions are summarized in Table 3. Additionally, data about recrystallization solvents, appearance (crystal form, color), molecular formula and elementary analysis for each compound or its acid addition salt are summarized in Table 4; and the IR- and NMR-spectrum data are shown in Table 5.

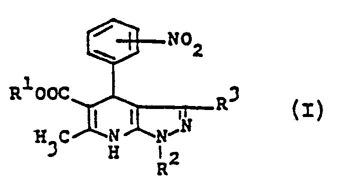

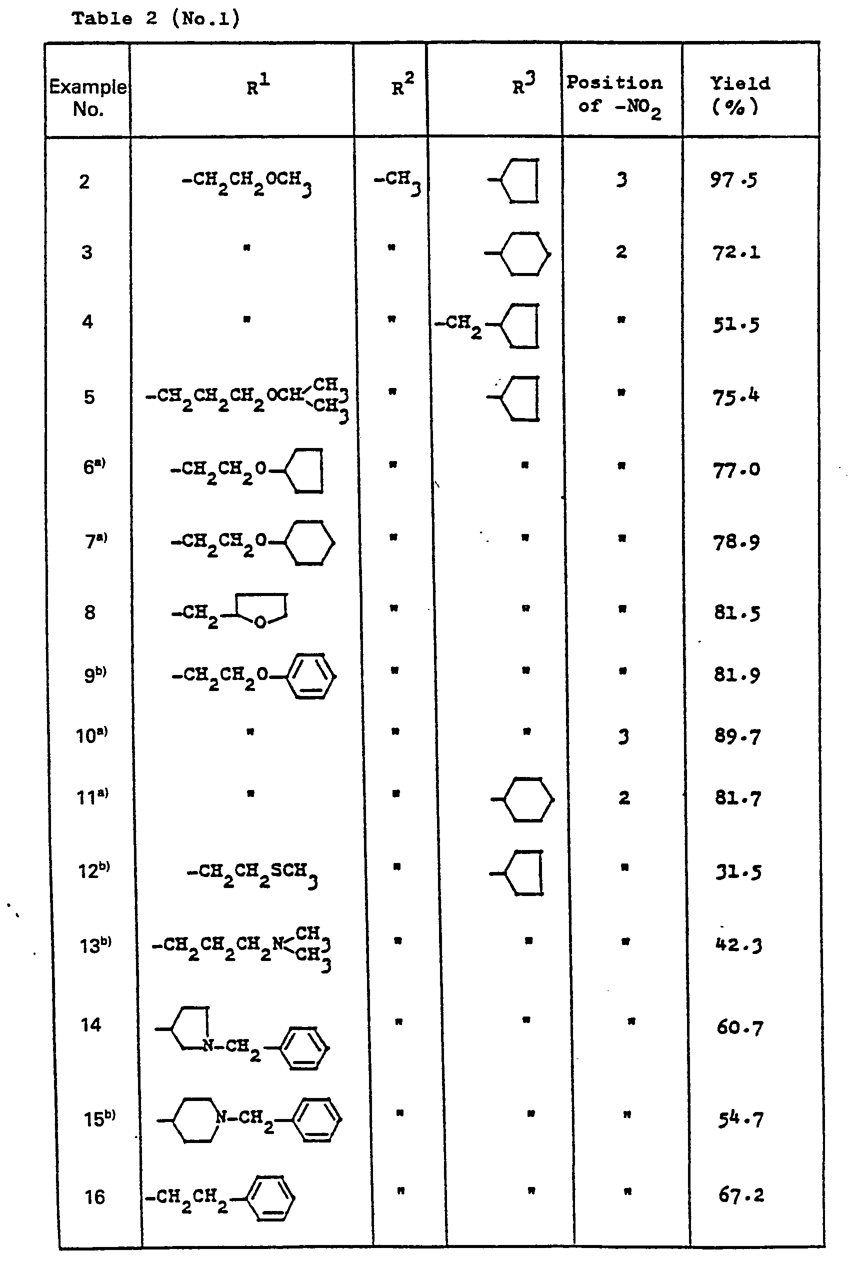

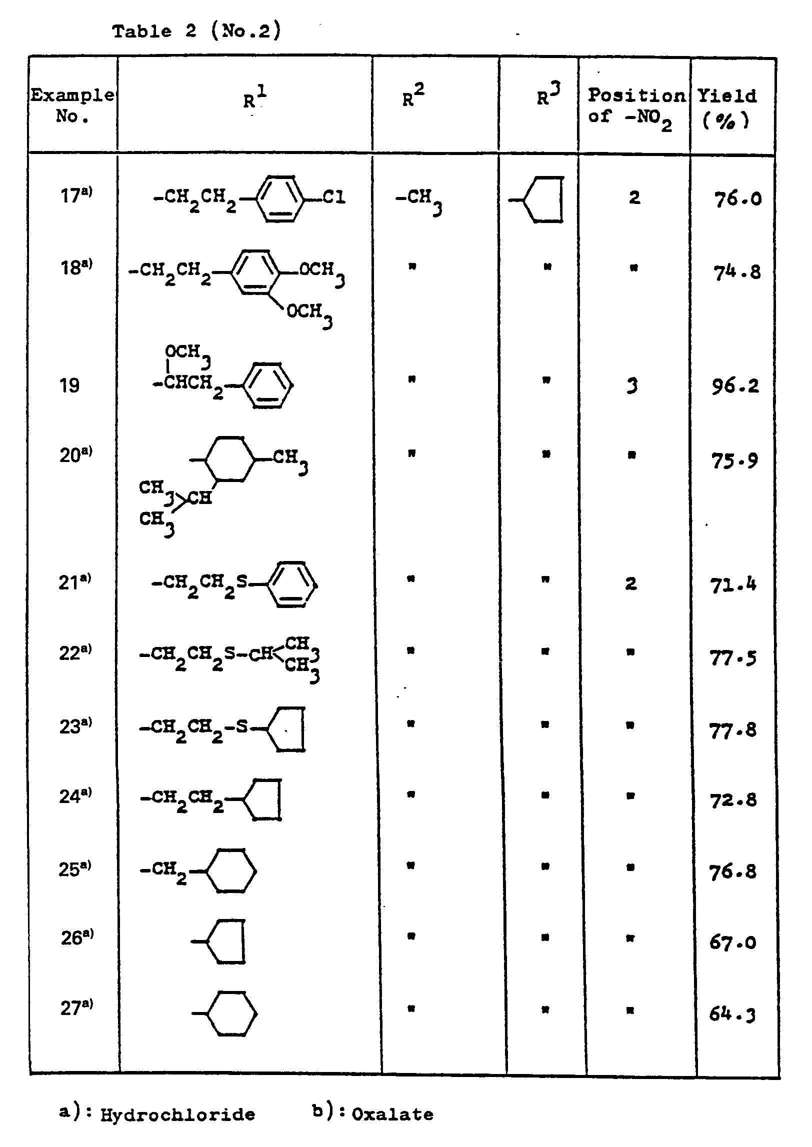

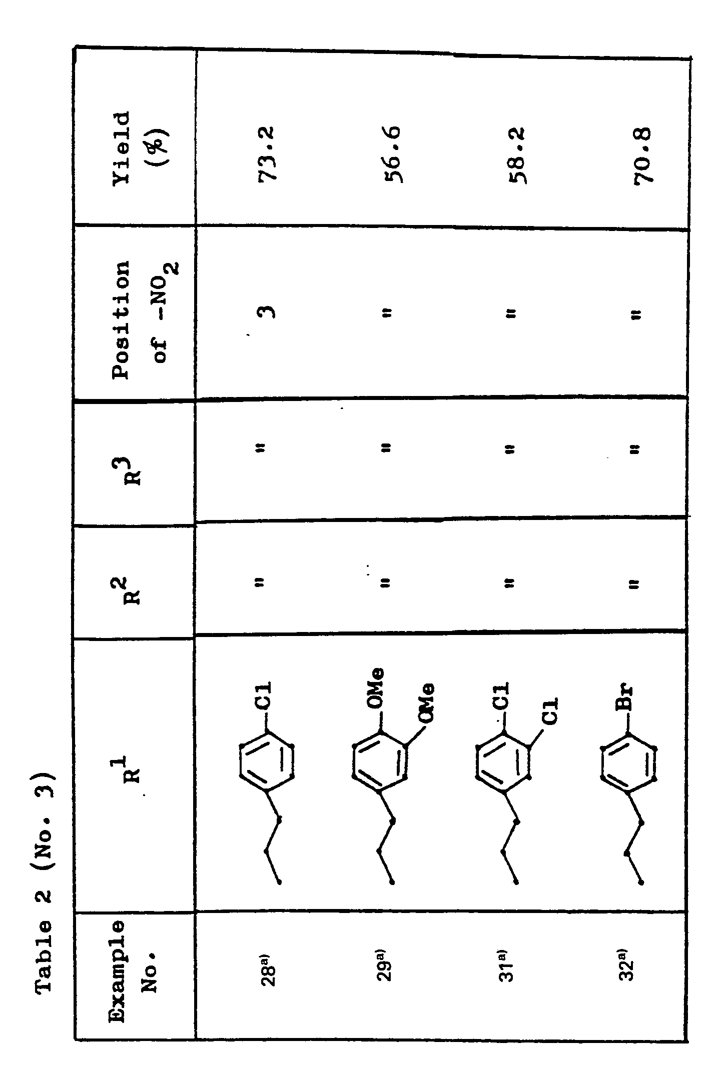

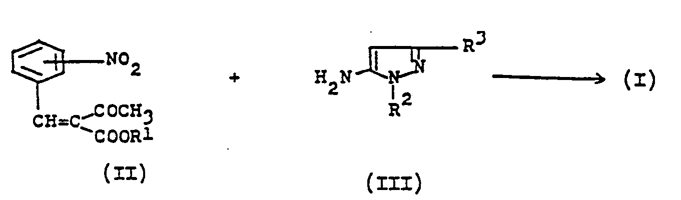

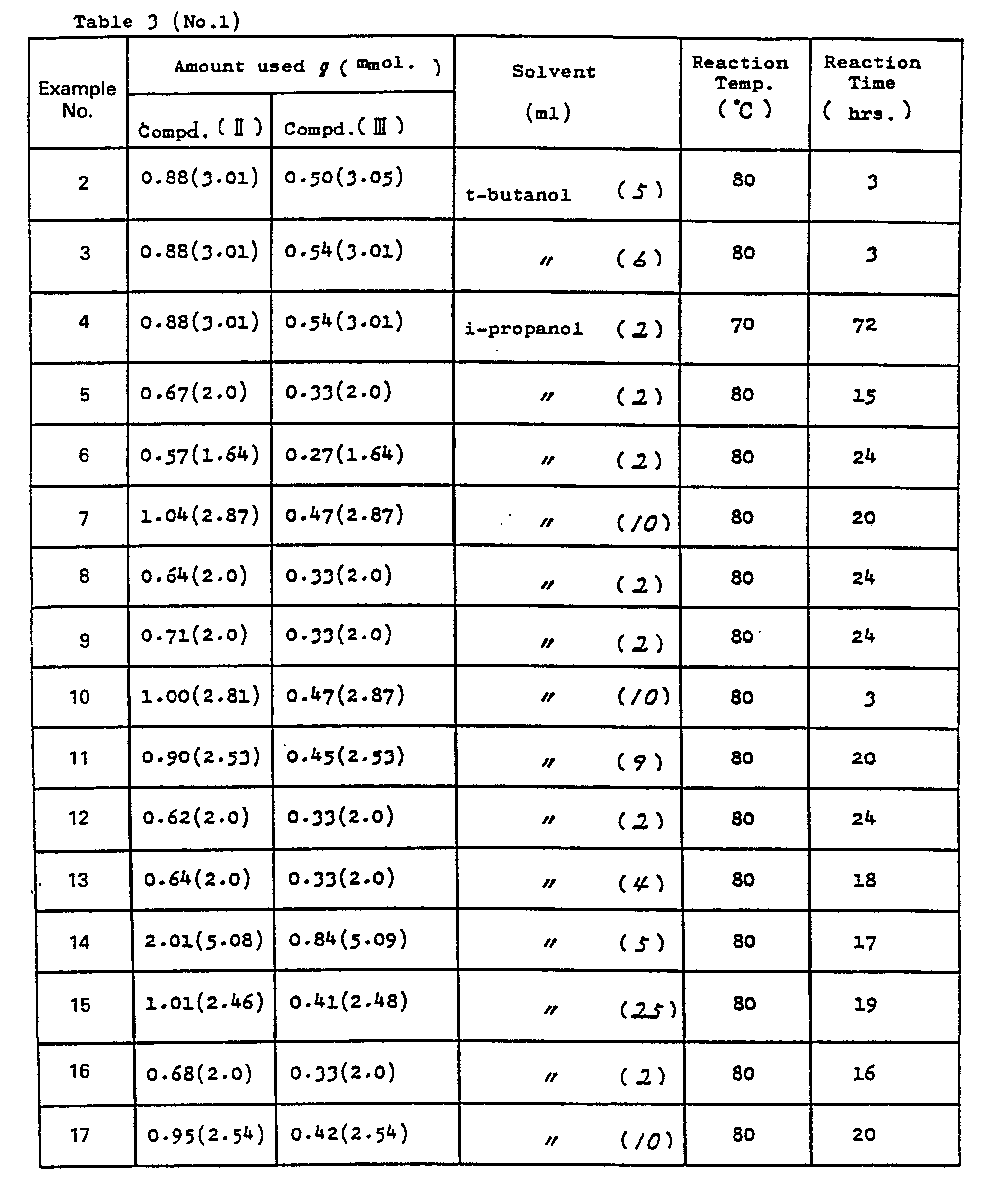

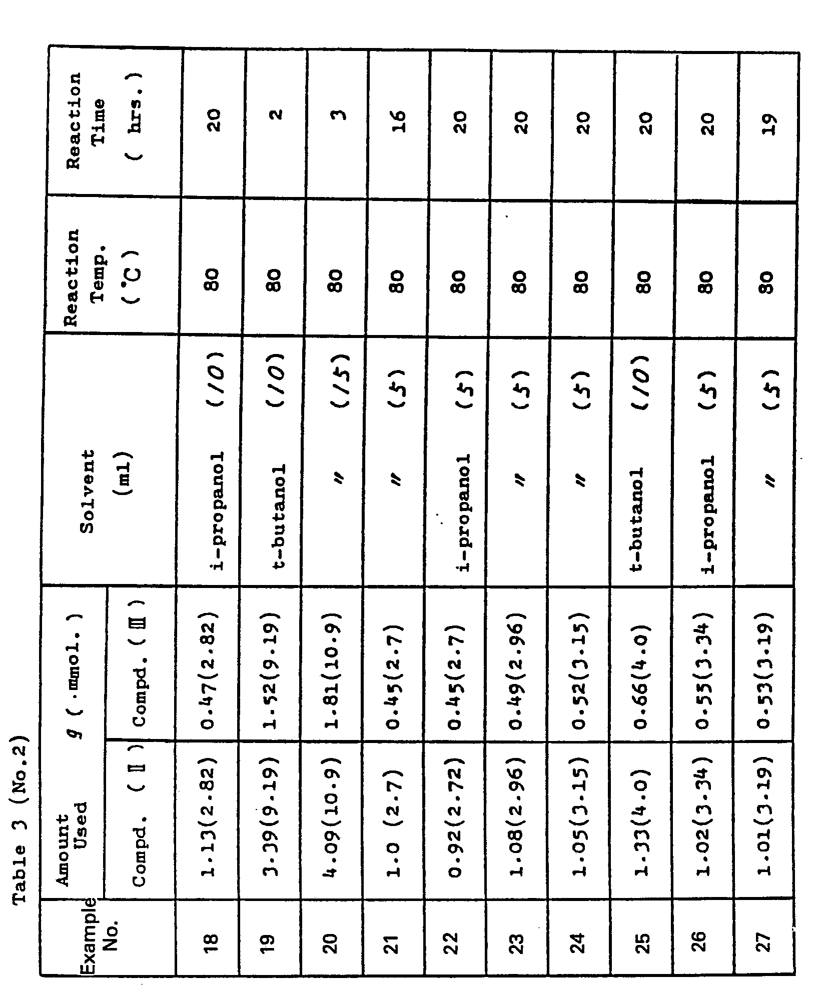

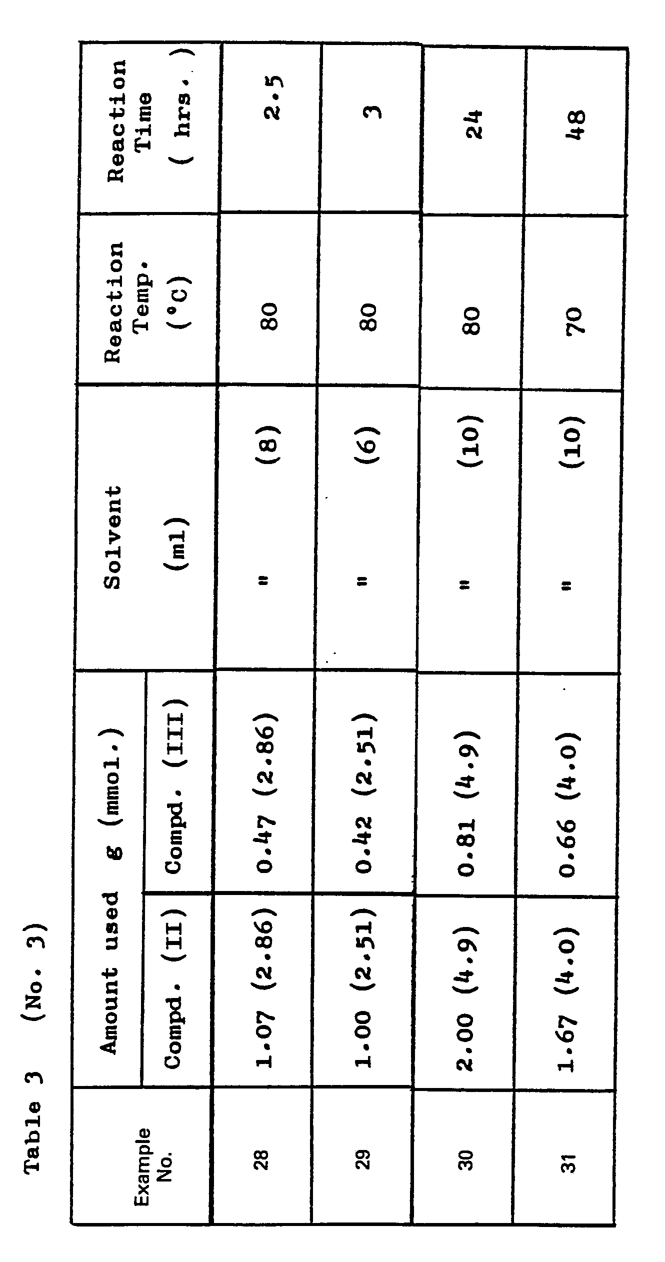

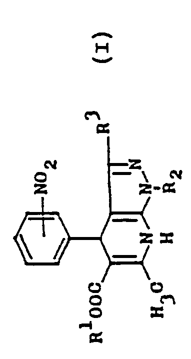

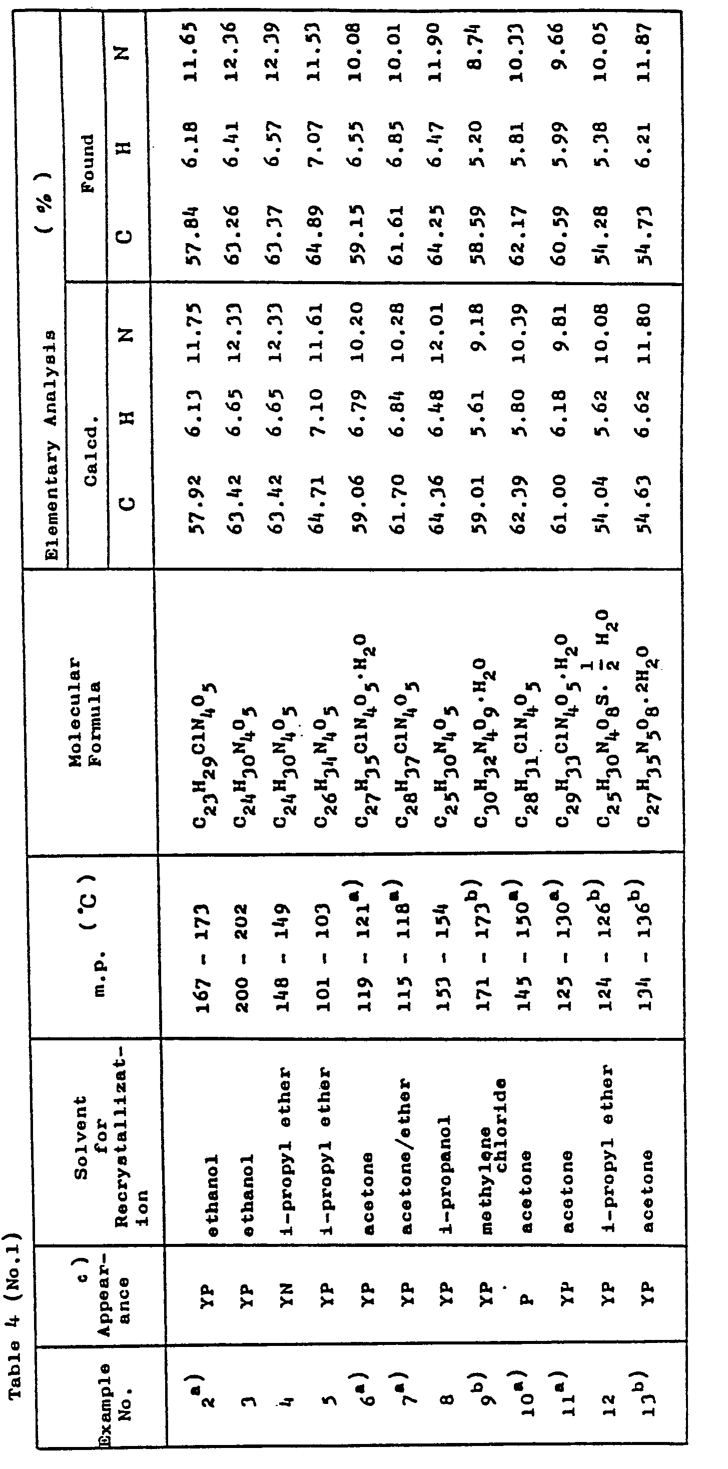

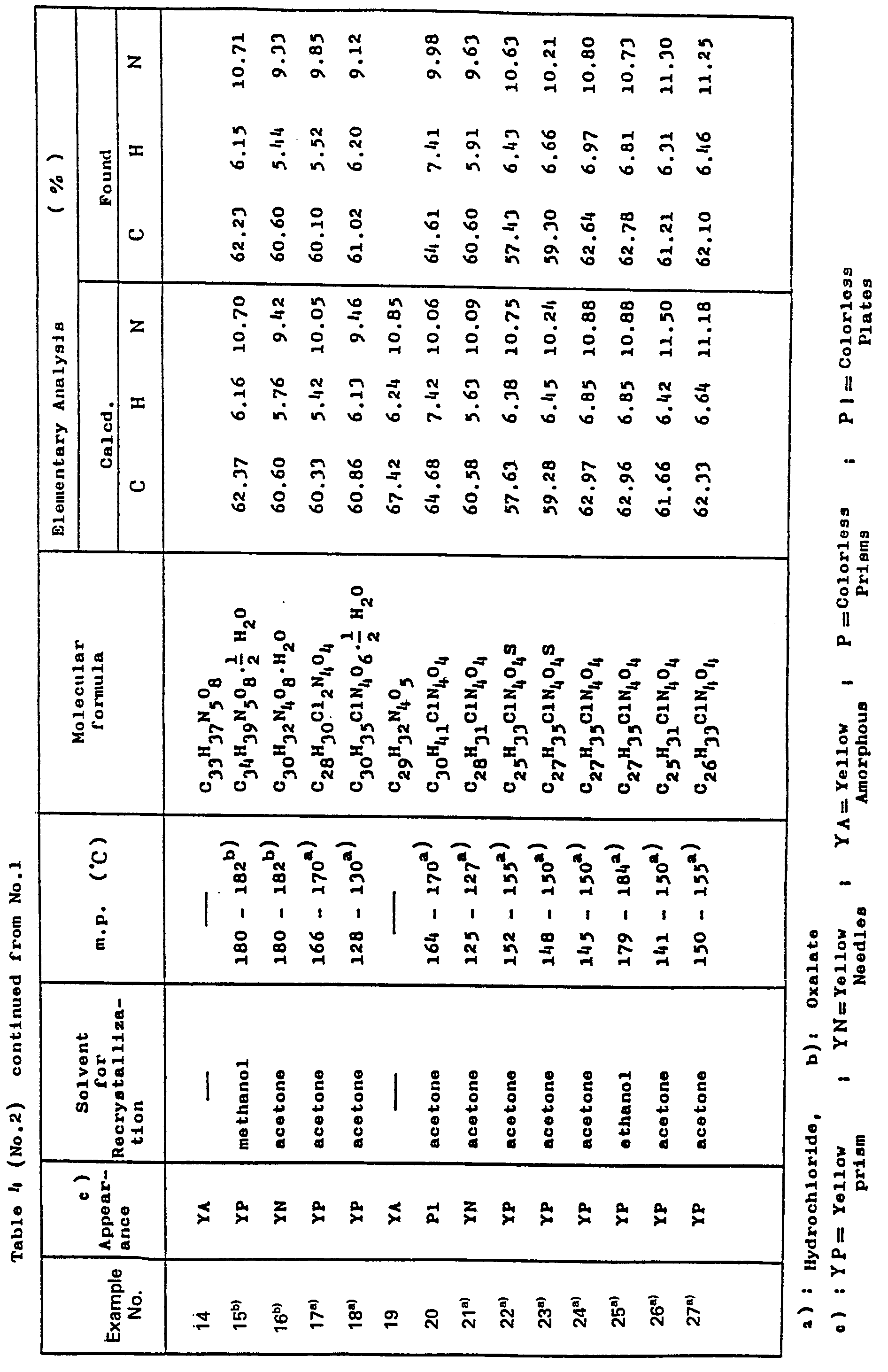

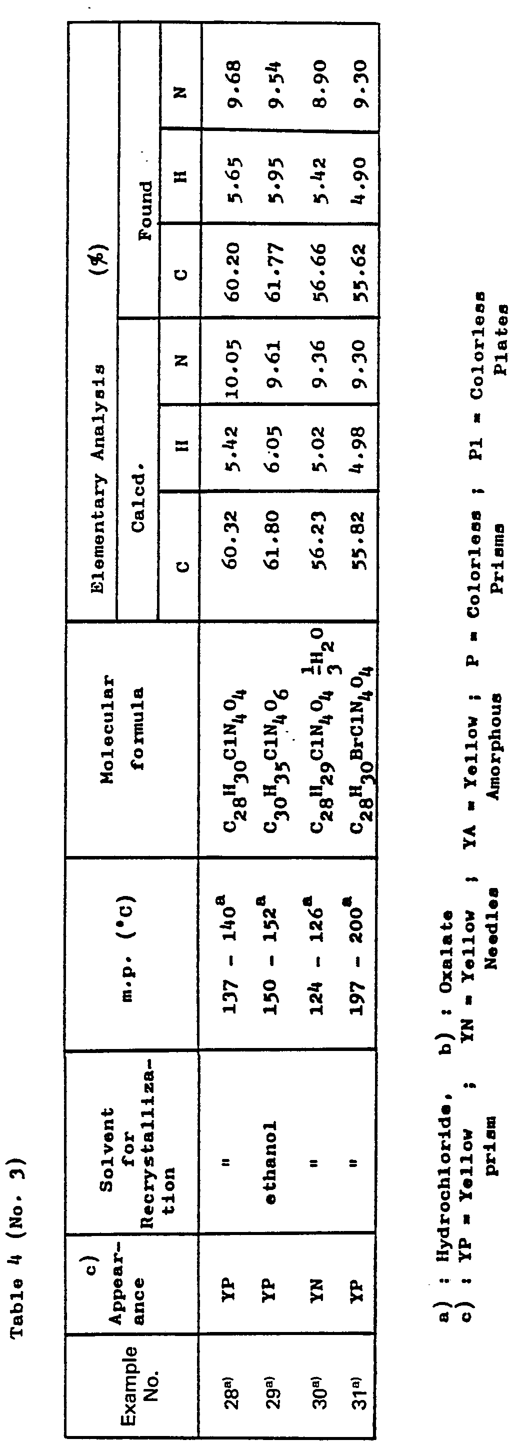

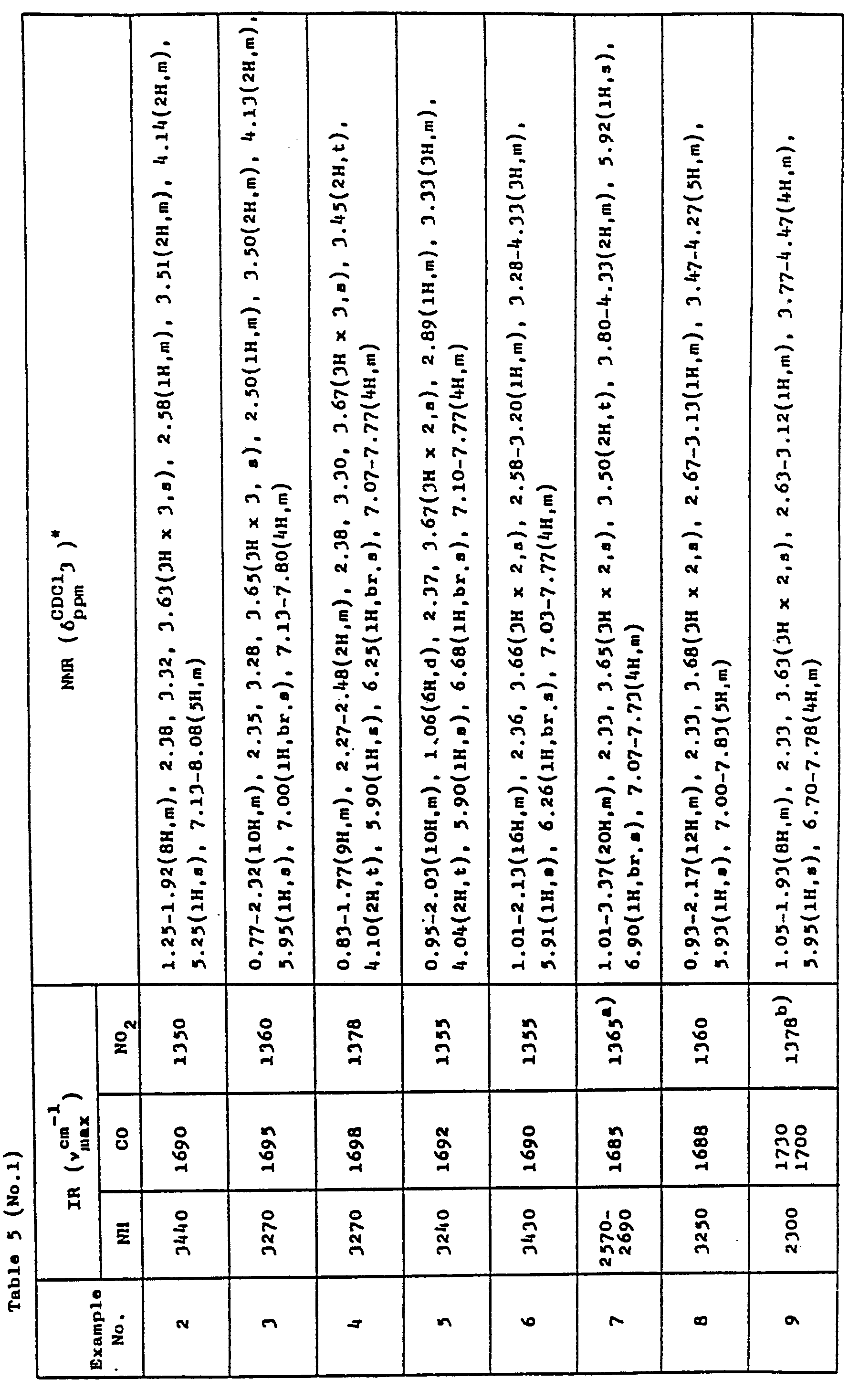

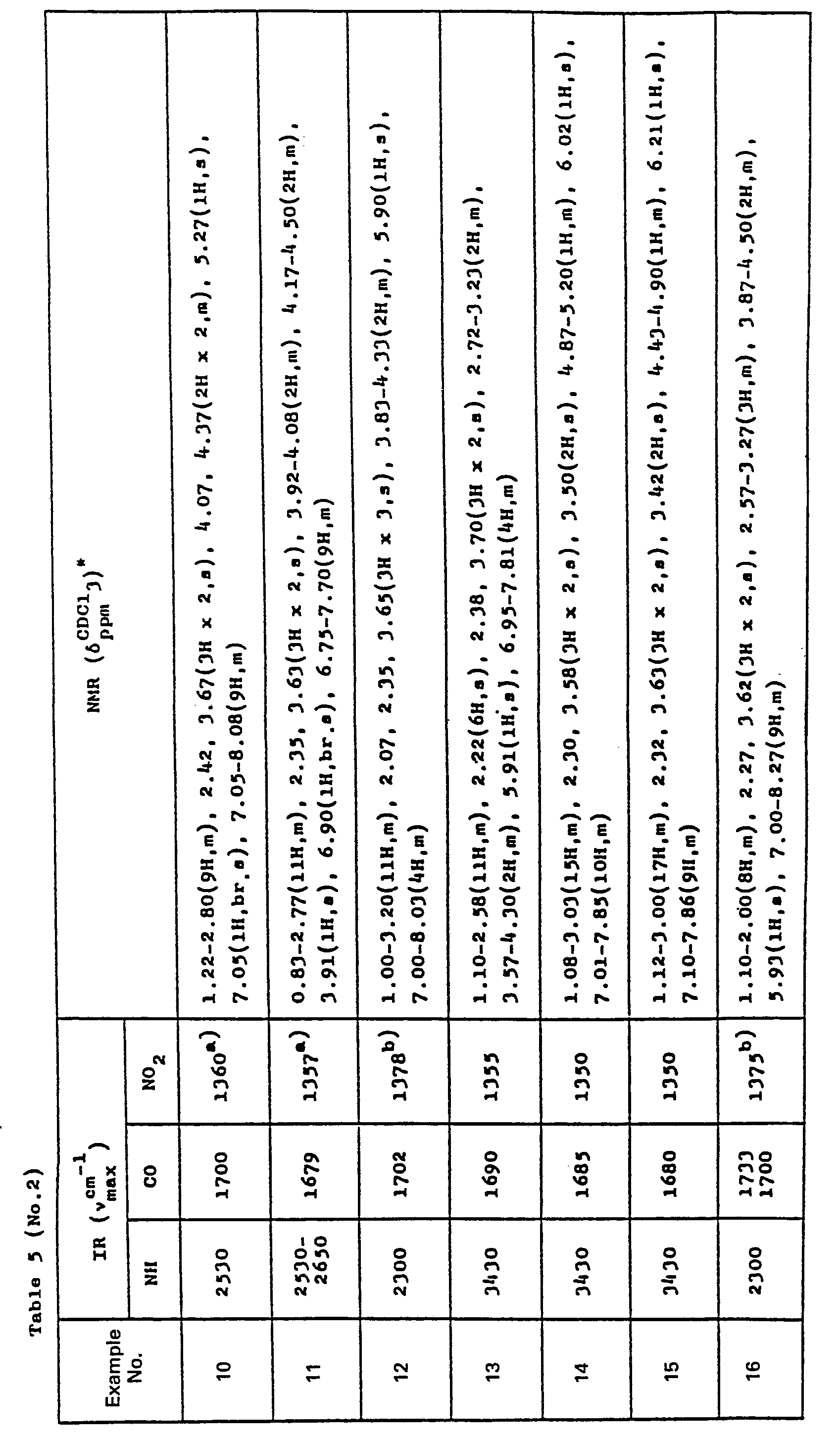

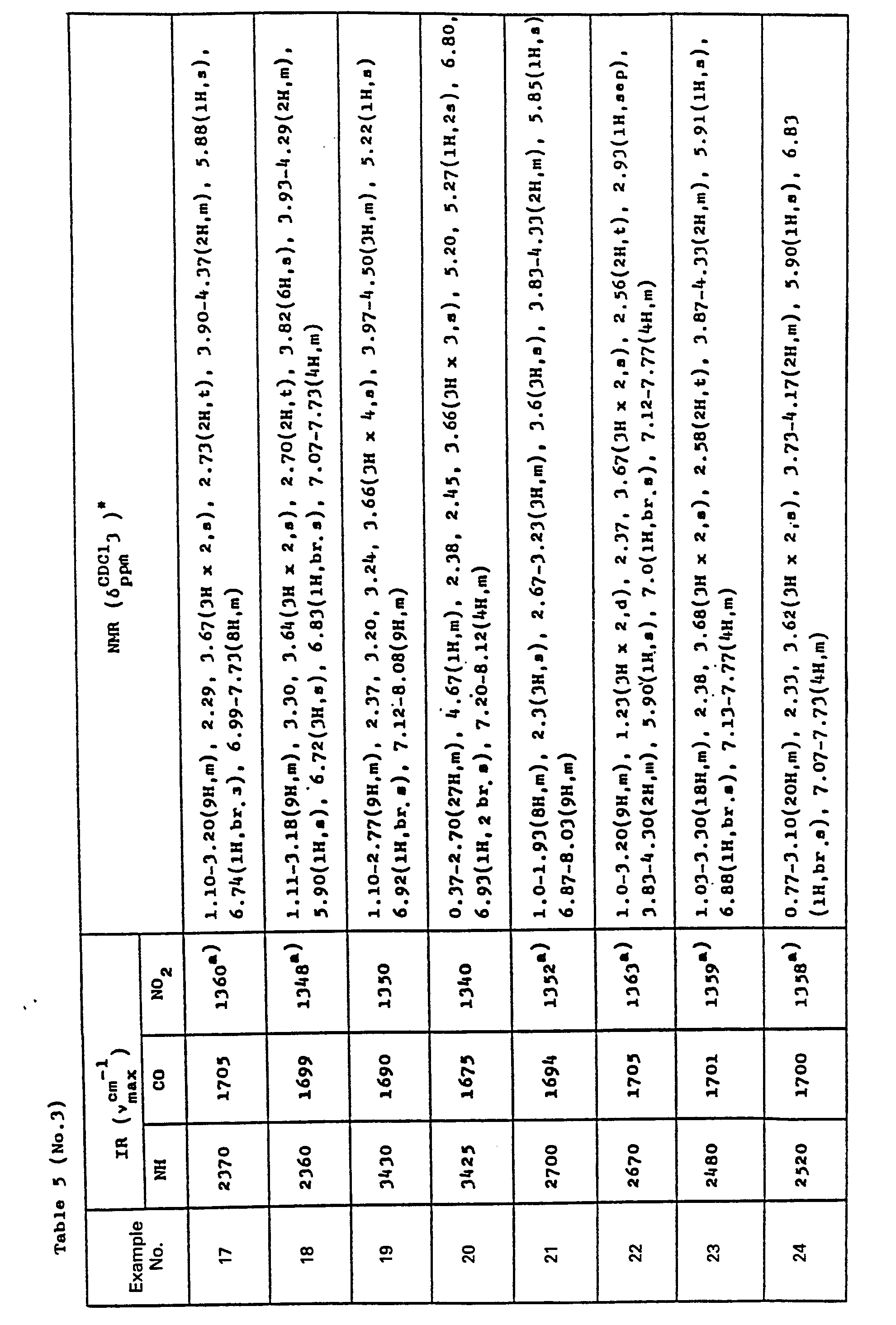

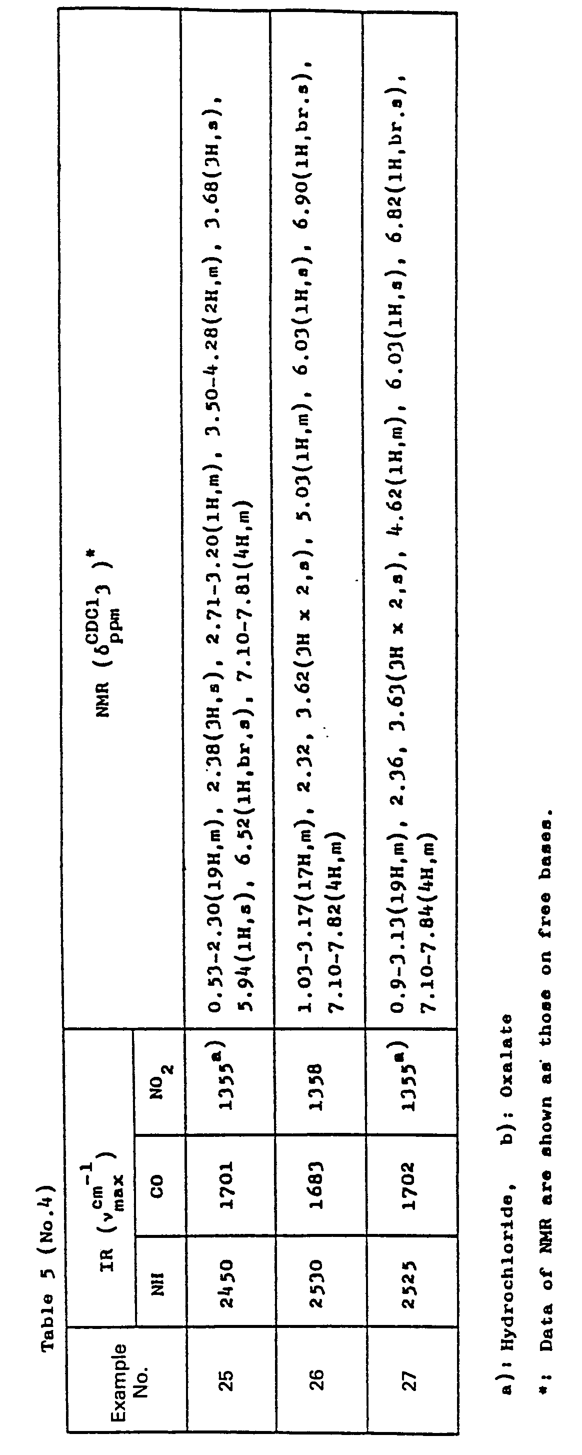

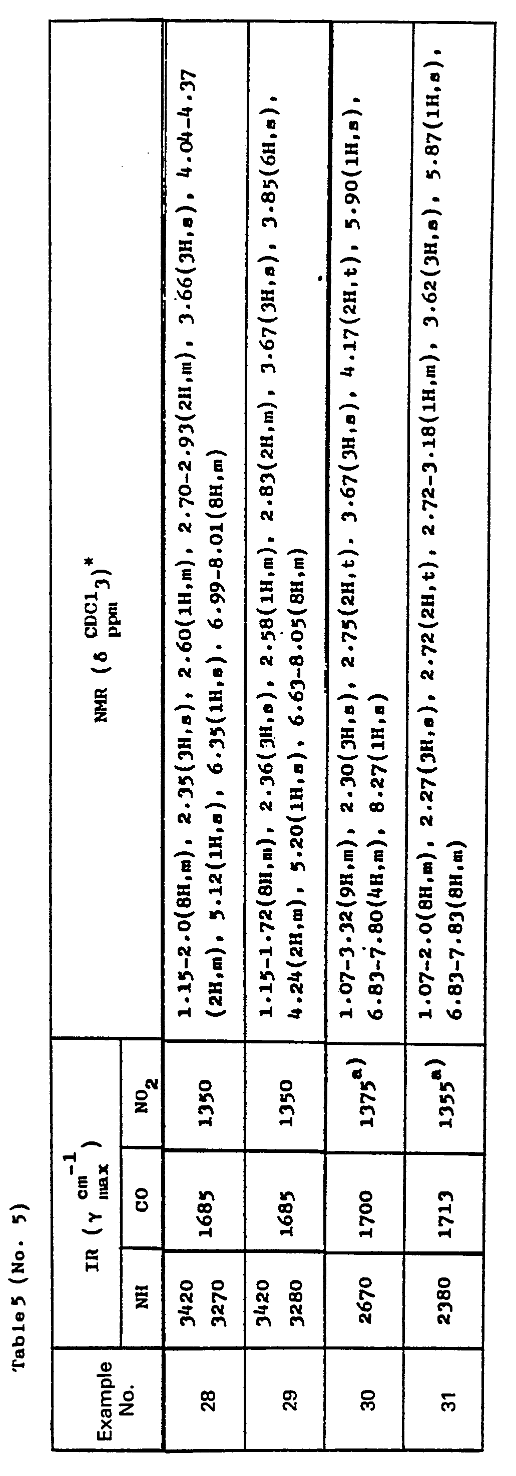

### Example 32

### Components for a tablet

Phenethyl 3-cyclopentyl-1,6-dimethyl-4-(2-nitrophenyl)-4,7-dihydropyrazolo[3,4-b]pyridine-5-carboxylate

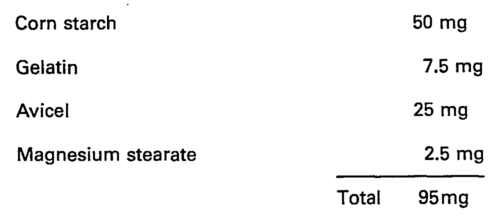

These substances are formulated into a tablet.

### Preparation 1

### Preparation of 2-methoxyethyl 2-nitrobenzylideneacetoacetate 6.

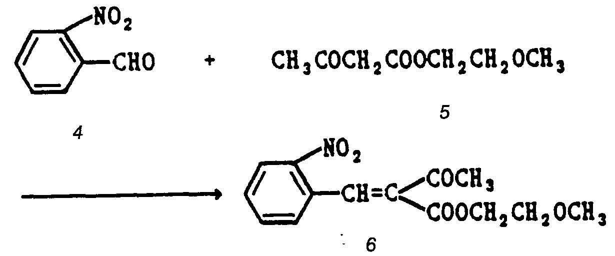

A solution of 4.80 g (30.0 mmol) of methoxyethylacetoacetate 5, 4.53 g (30.0 mmol) of 2-nitrobenzaldehyde 4, 1.0 ml of acetic acid and 0.3 ml of piperidine in 25 ml of benzene is stirred at room temperature for 17 hours, and the mixture is refluxed for 2 hours under conditions of azeotropic distillation. The reaction mixture is washed with water, dried over magnesium sulfate, filtered, and evaporated. The residue is chromatographed on silica gel for purification to give 7.93 g (90.2% yield) of the title compound 6 as a yellow oil [from the eluate with dichloromethane/ethyl acetate (95/5)].

NMR(CDCI₃) 6ppm: 2.23, 2.48(3H, 2s), 3.17-3.80(2H, m), 3.22, 3.40(3H, 2s), 4.08―4.53(2H, m), 7.33―8.33(5H, m).

### Preparations 2-29

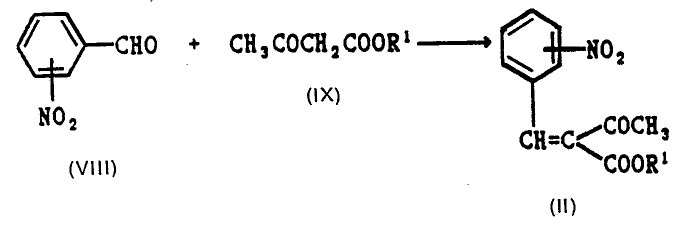

wherein R' has the same meaning as defined above.

A solution of the compound (VIII) and a compound (IX) in a solvent is allowed to react in the presence of an organic base at room temperature or under heating, and then refluxed if needed. The reaction mixture is washed with water, dried, filtered, and evaporated. According to requisition, the resulting residue is subjected to recrystallization or chromatography on silica gel for purification purposes.

Each of the compounds (II) listed in the following Table 6 can be prepared in the above-depicted manner. The yield for a compound (II) is listed in Table 6. The Process for production of the starting substances (IX) is depicted on top of Table 6, and the yield thereof is also listed in Table 6. In addition thereto, details of the reaction conditions for the above reaction (Preparations 2-25) are summarized in Table 7. Boiling and melting points and NMR spectrum data for each of the compounds (II) and the starting substances (IX) are summarized in Table 8.

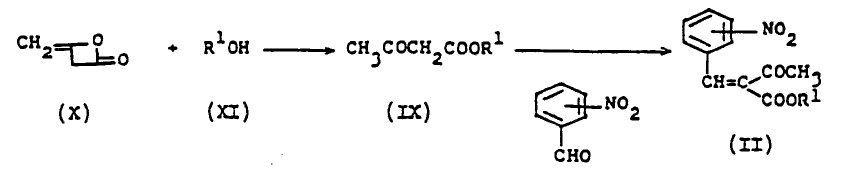

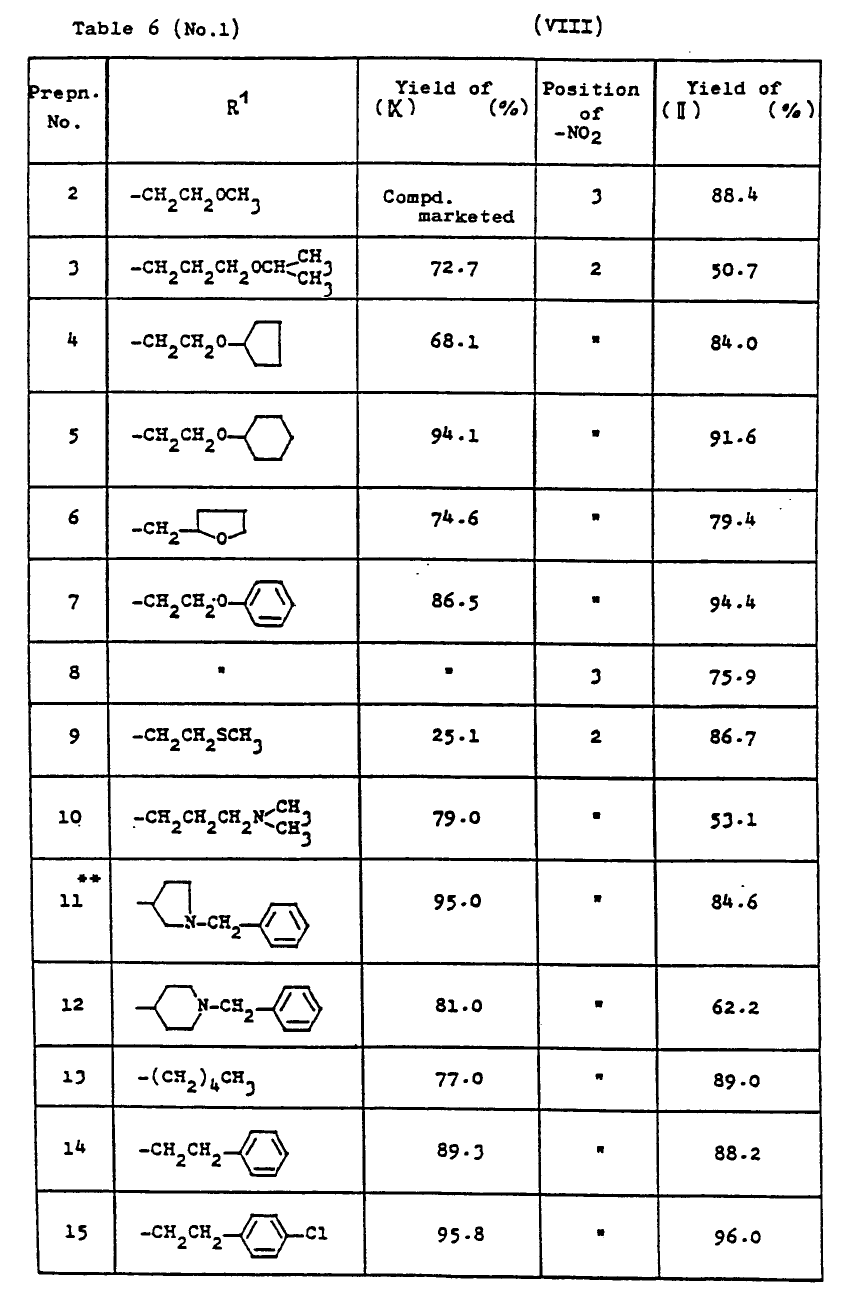

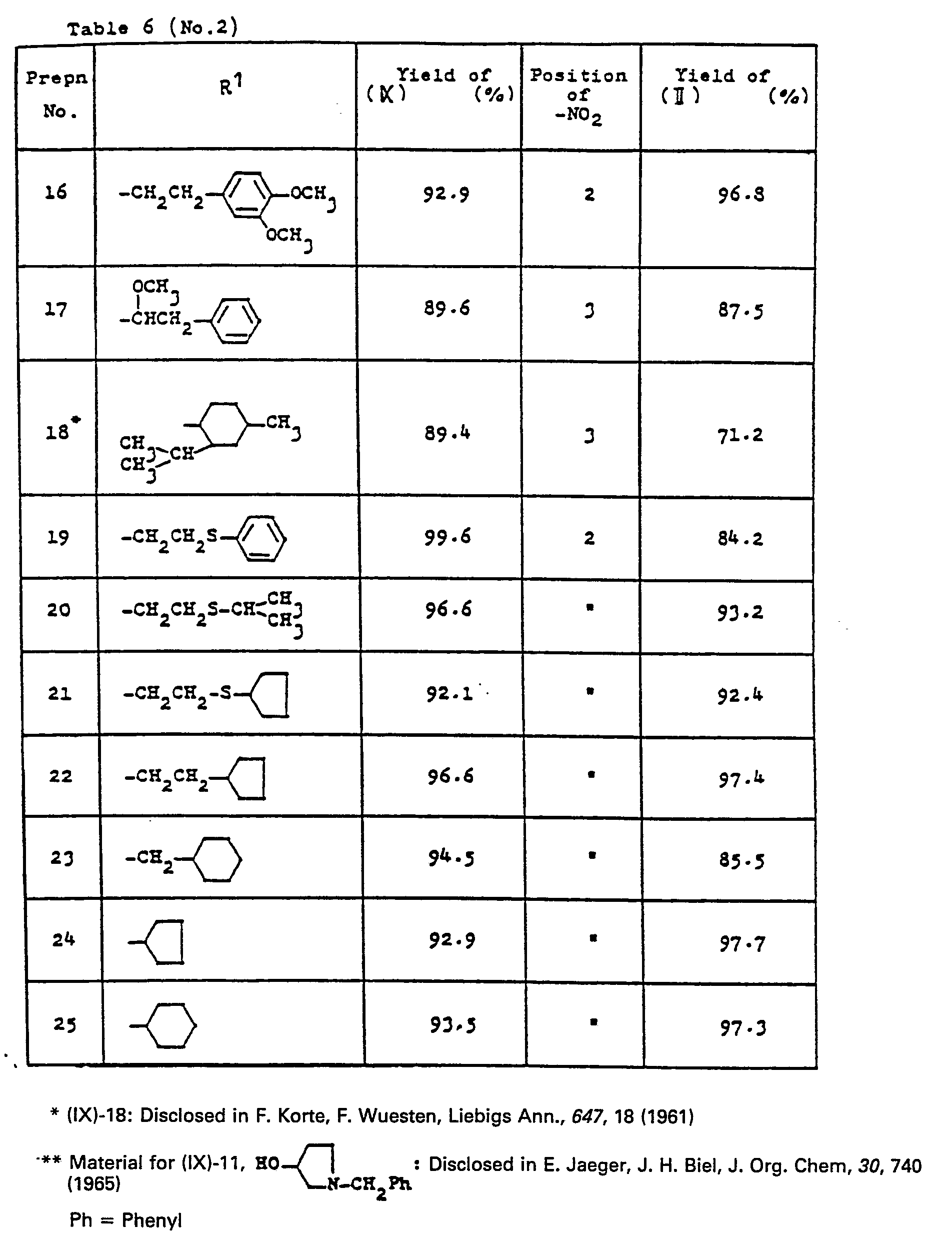

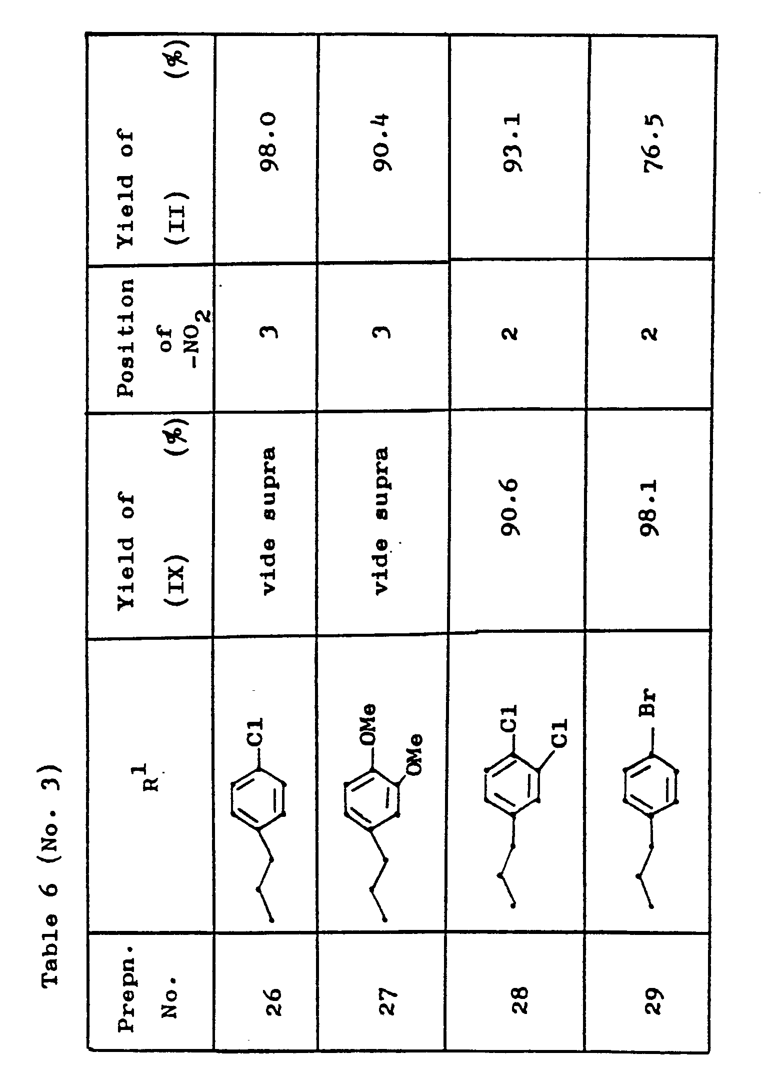

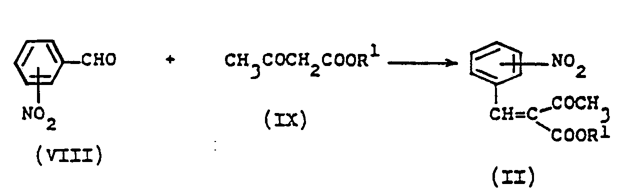

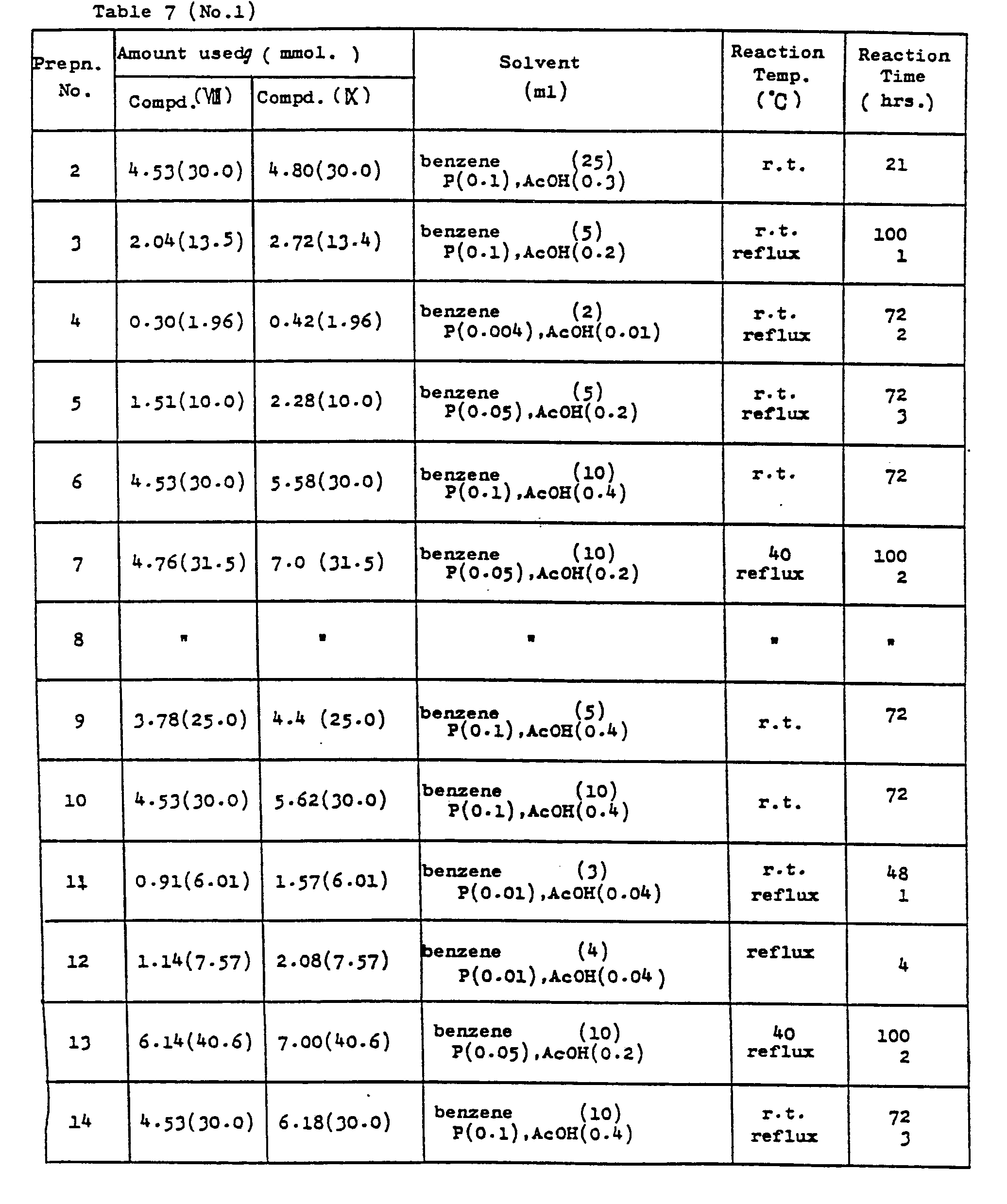

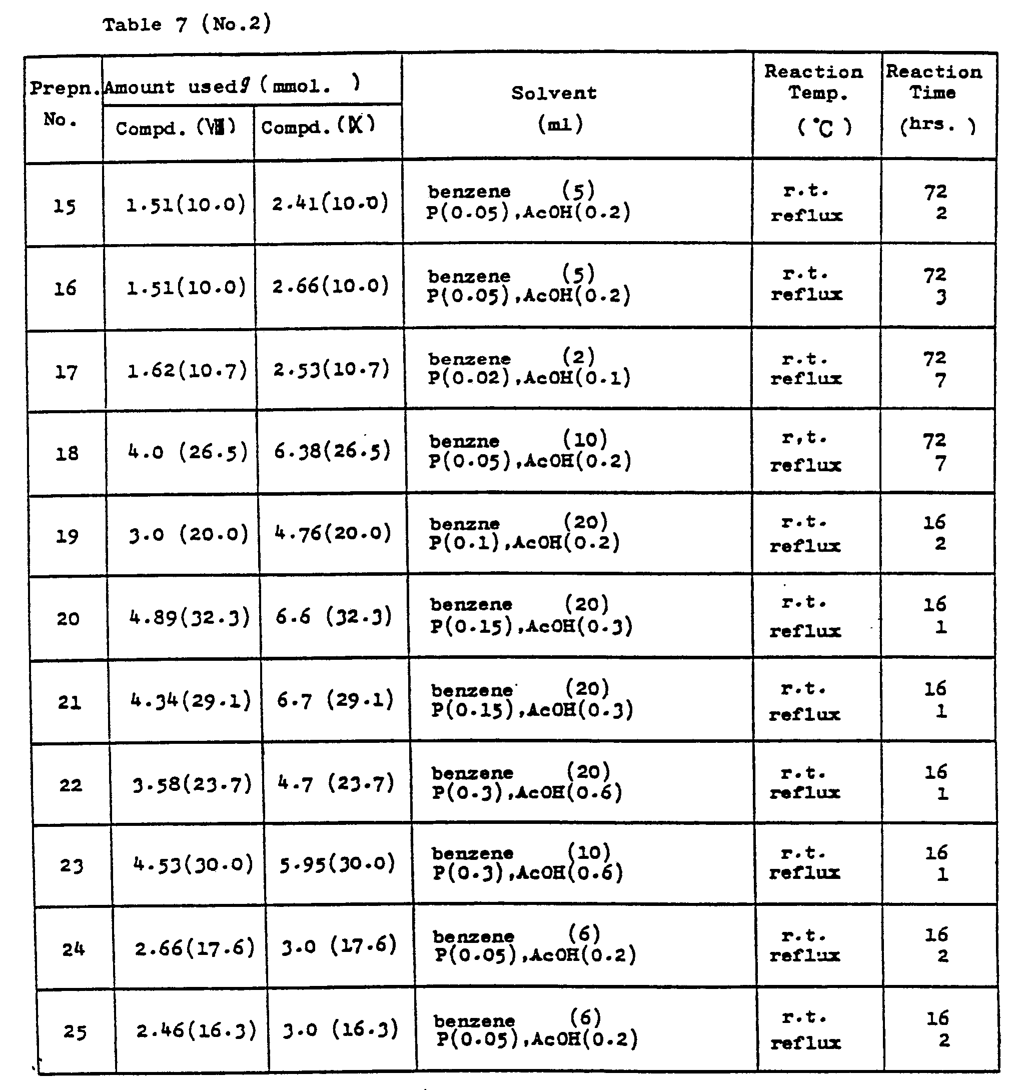

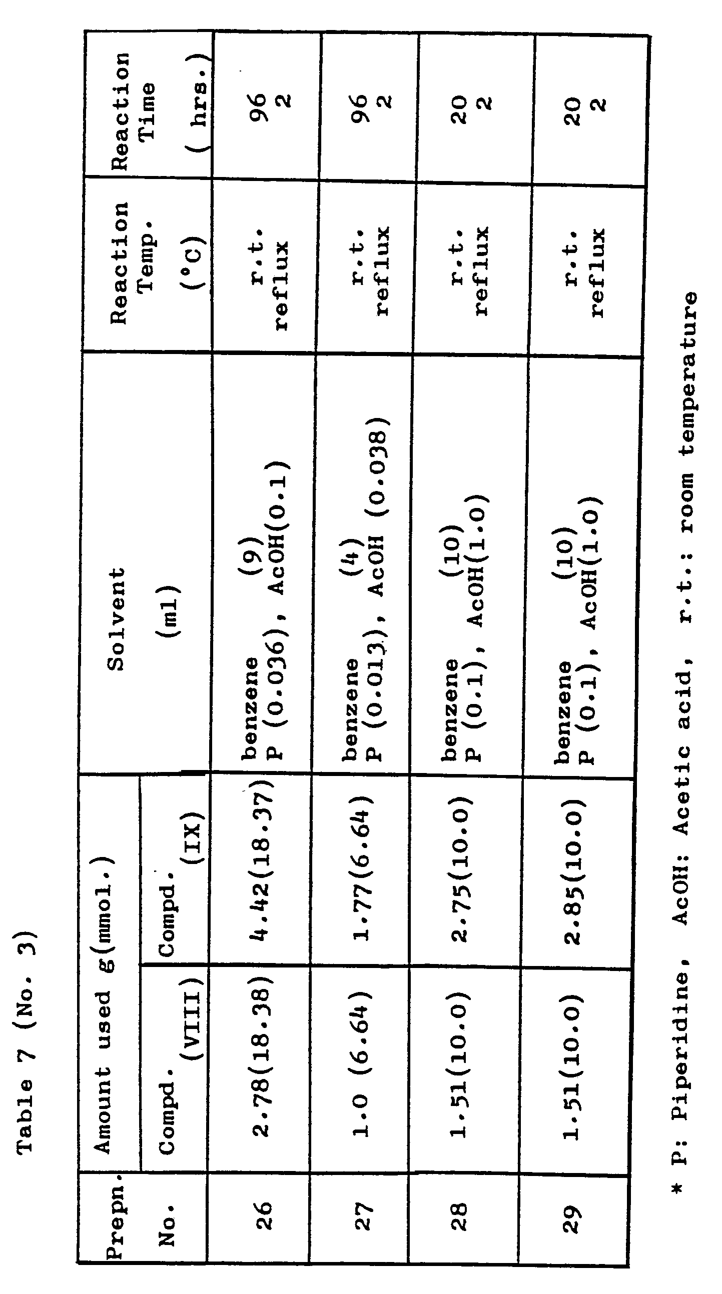

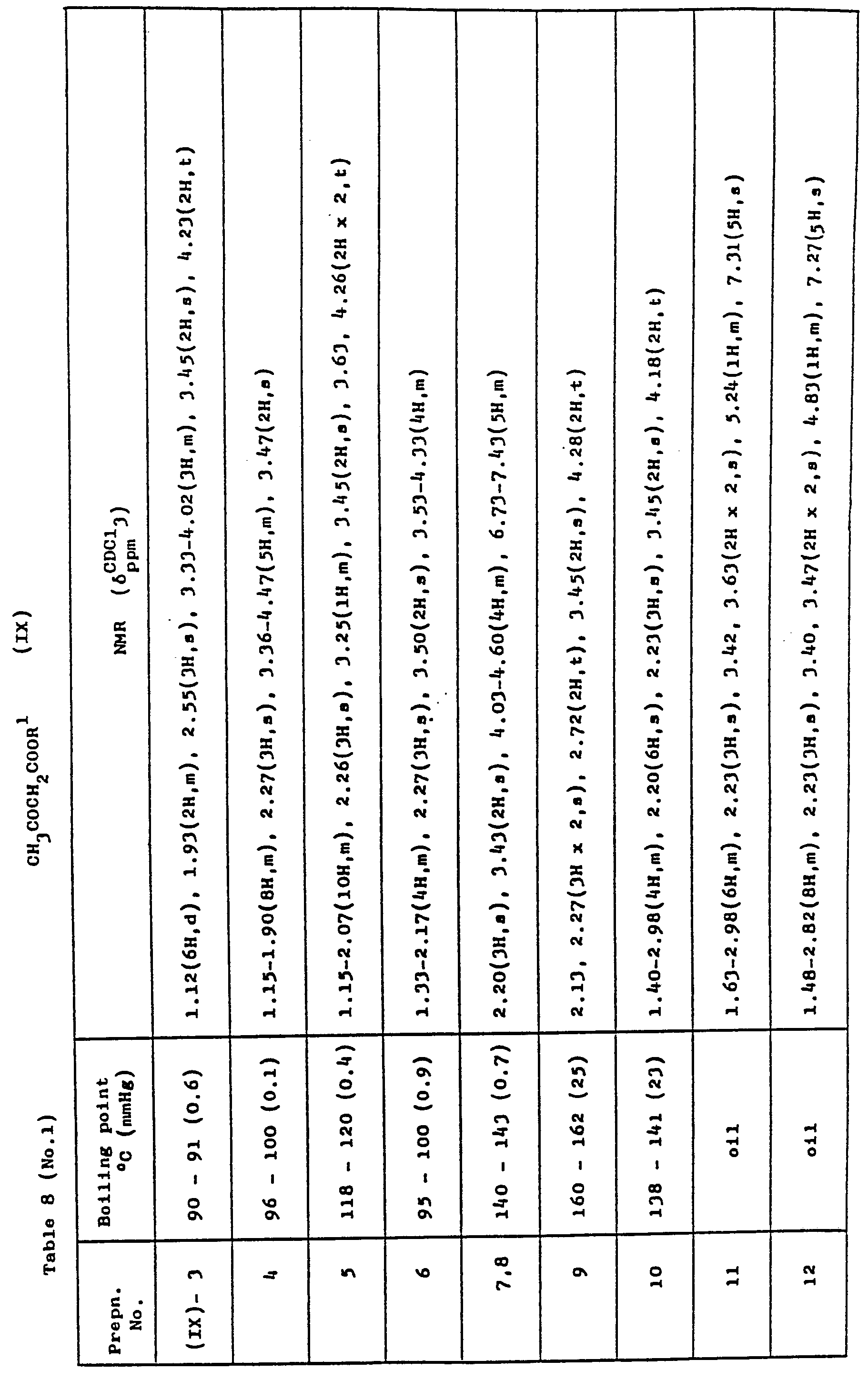

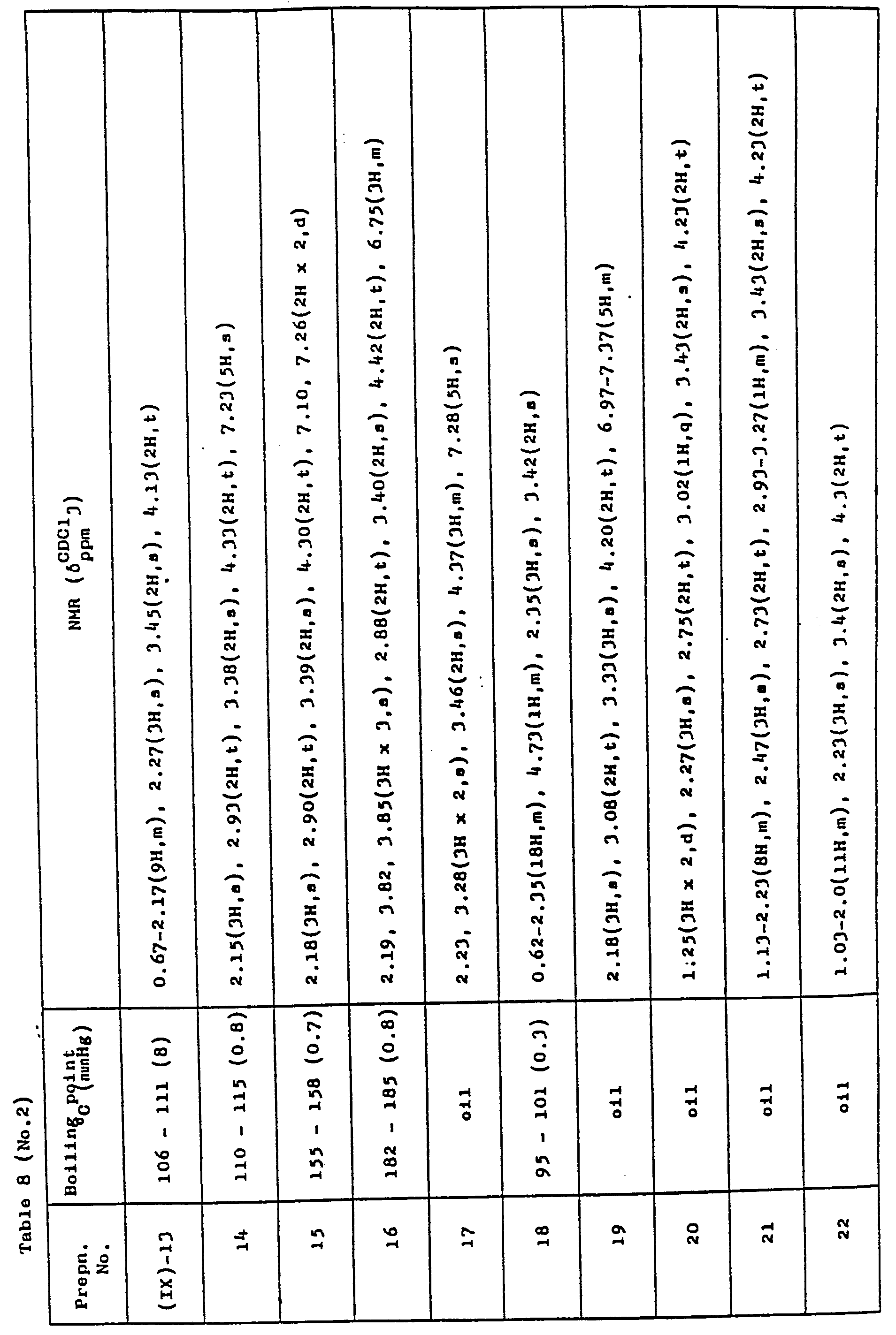

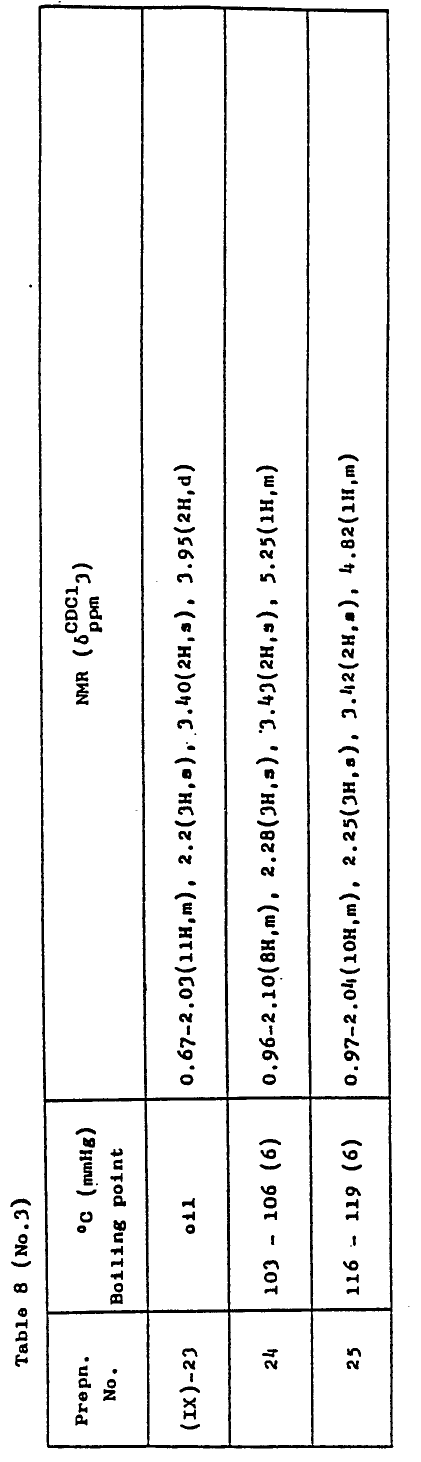

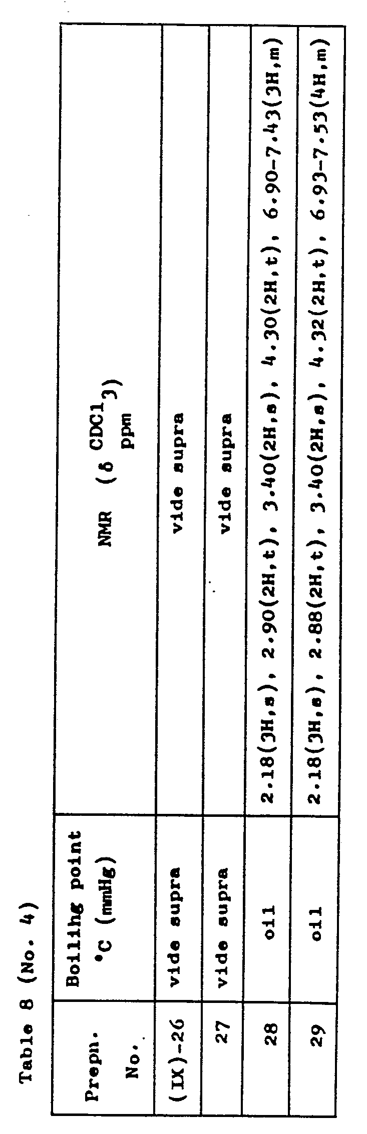

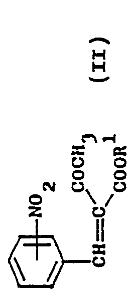

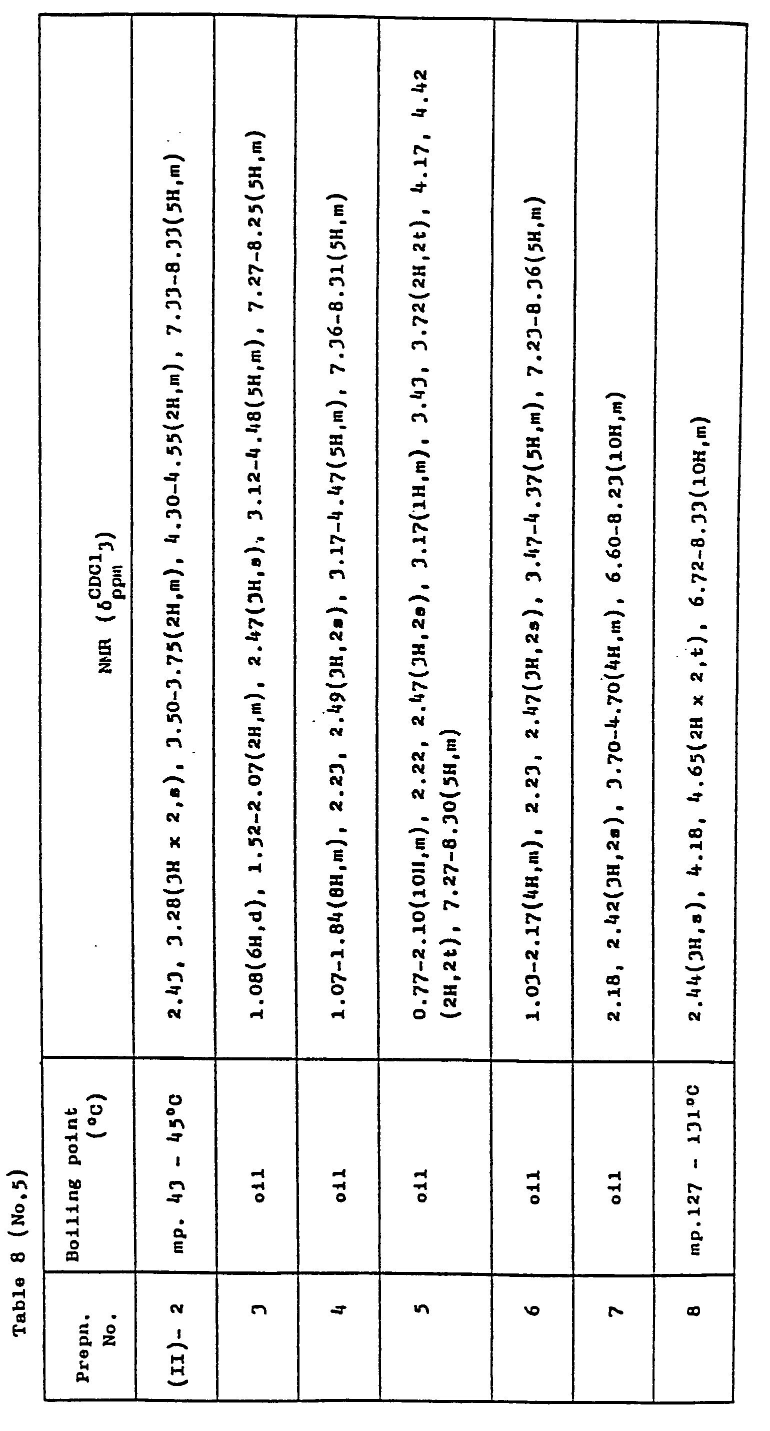

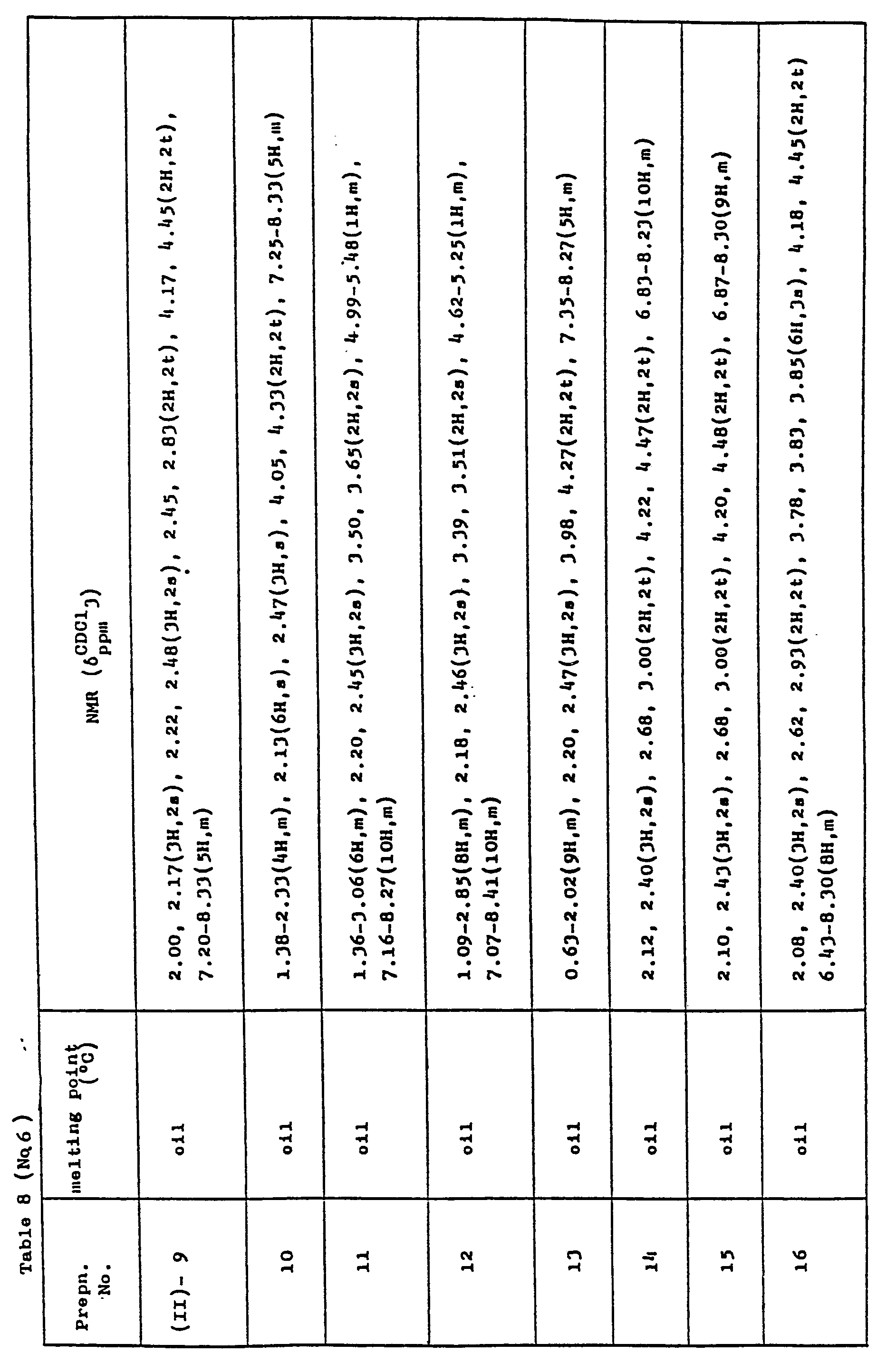

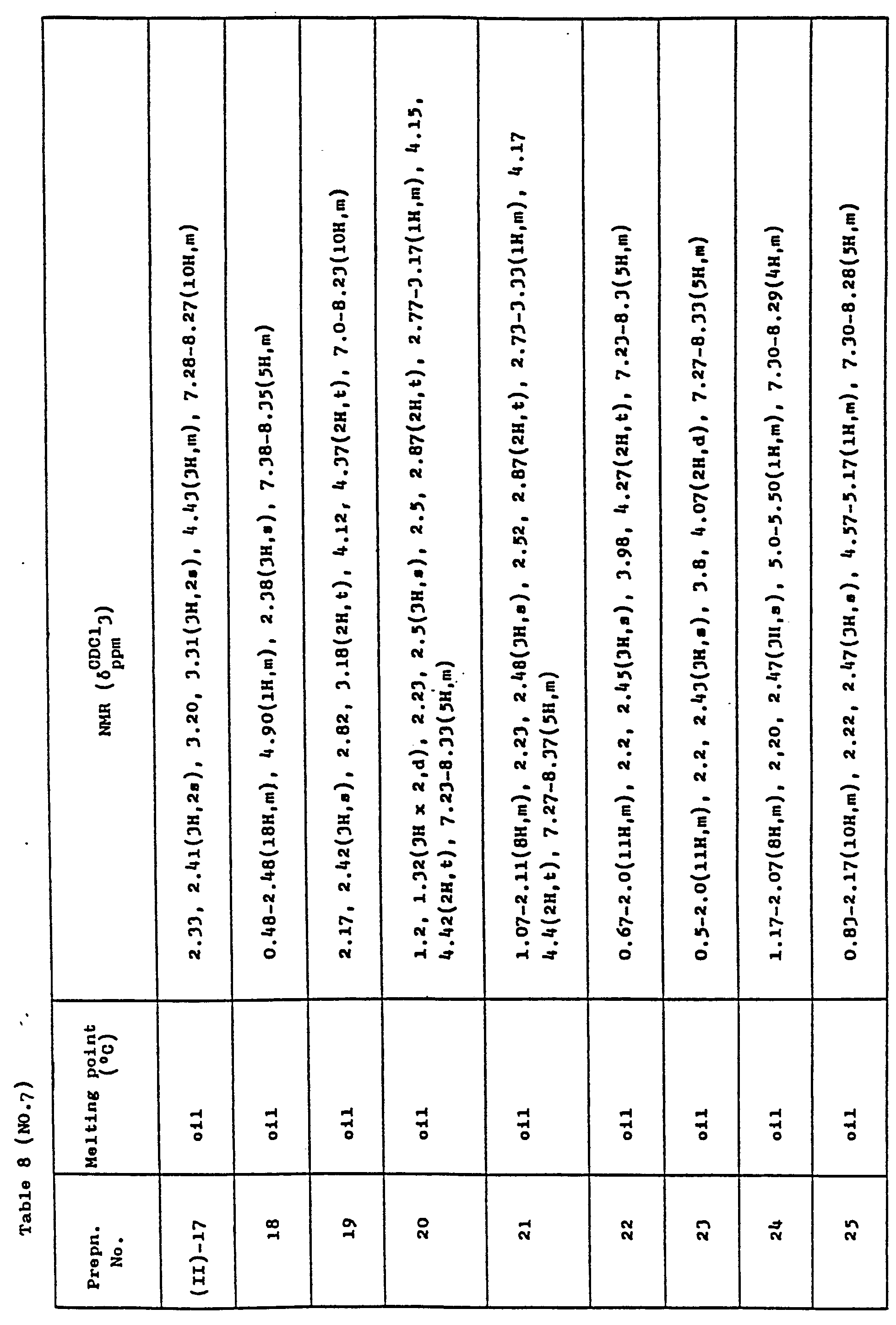

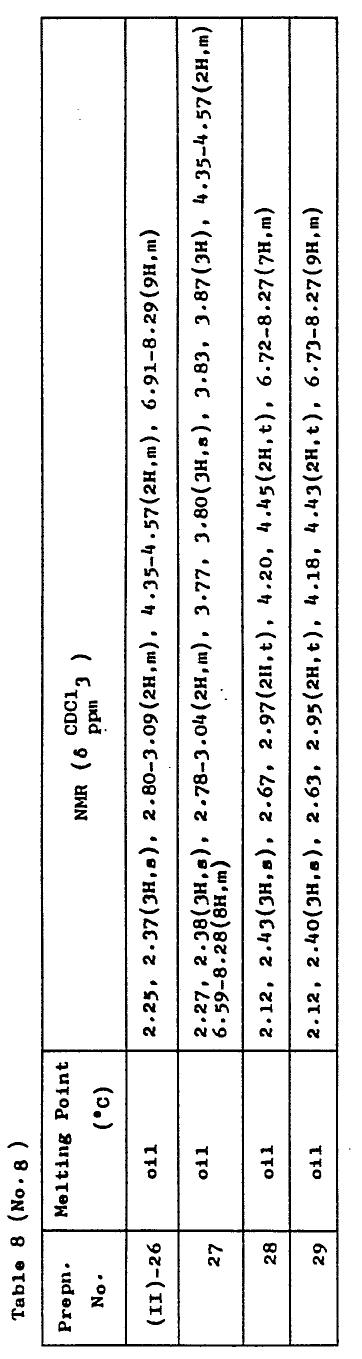

### Preparation 30

### (i) Preparation of 3-cyclopentylcarbonylacetonitrile 9.

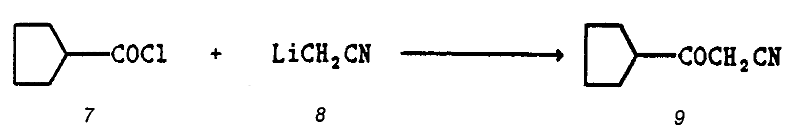

To 20 ml oftetrahydrofuran is added 15.6 ml (26 mmol) of butyl lithium in an atmosphere at -78°C, and 1.3 g (32 mmol) of acetonitrile is added dropwise to the mixture over a period of 40 minutes. Two hours later, 3.43 g (25.9 mmol) of cyclopentylcarbonyl chloride 7 is further added dropwise over a period of 40 minutes. The reaction mixture is, after being stirred for 2 hours, neutralized with 10% hydrochloric acid and extracted with ether. The extract solution is dried over magnesium sulfate, filtered, and then evaporated. The resulting residue is subjected to column chromatography to give 2.91 g (80.8% yield) of the title compound 9 as an oil (from the chloroform layer).
NMR(CDCI₃) δppm: 1.43-2.2 (8H, m), 2.7-3.33 (1H, m), 3.53 (2H, s).

### (ii) Preparation of 5-amino-3-cyclopentyl-1-methylpyrazole 11.

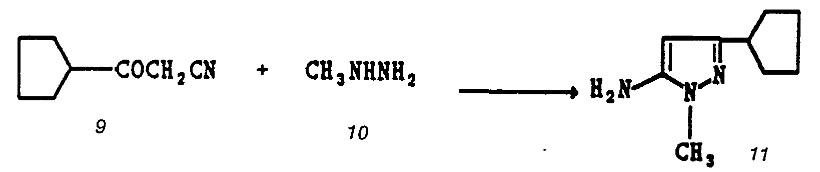

To a solution of 13.21 g (96.3 mmol) of 3-cyclopentylcarbonylacetonitrile 9 in 10 ml of dioxane is added 4.44 g (96.3 mmol) of methylhydrazine 10 under ice-cooling, and the mixture is stirred at room temperature for 17 hours. The reaction mixture is evaporated. The resulting residue is recrystallized from ethyl acetate/ hexane (5/2) to give 12.9 g (80.8% yield) of the title compound 11 as colorless needles, mp.149-150°C.
NMR(CDCI₃) 5ppm: 1.33-2.20 (8H, m), 2.60-3.10 (1H, m), 3.57 (5H, s), 5.32 (1H, s).

### Preparations 31 and 32

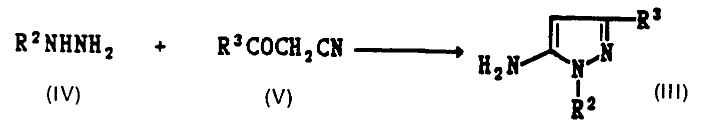

wherein R² and R³ have the same meanings as defined above.

A solution of compounds (IV) and (V) in a solvent is allowed to react at room temperature, then evaporated, and the resulting residue is purified by recrystallization, if needed.

In the above-depicted manner, the compounds (III) can be prepared. Details of the reaction conditions are summarized in Table 9, and data about yield, melting points, and N MR spectrum of the compounds are given in Table 10.

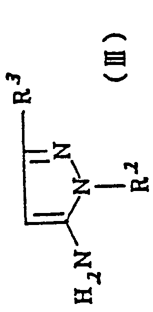

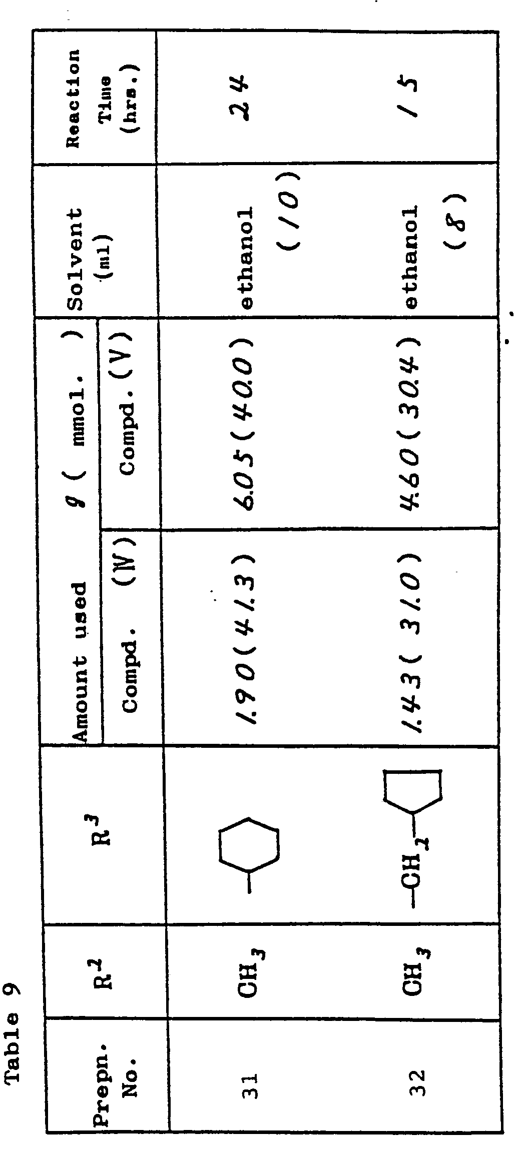

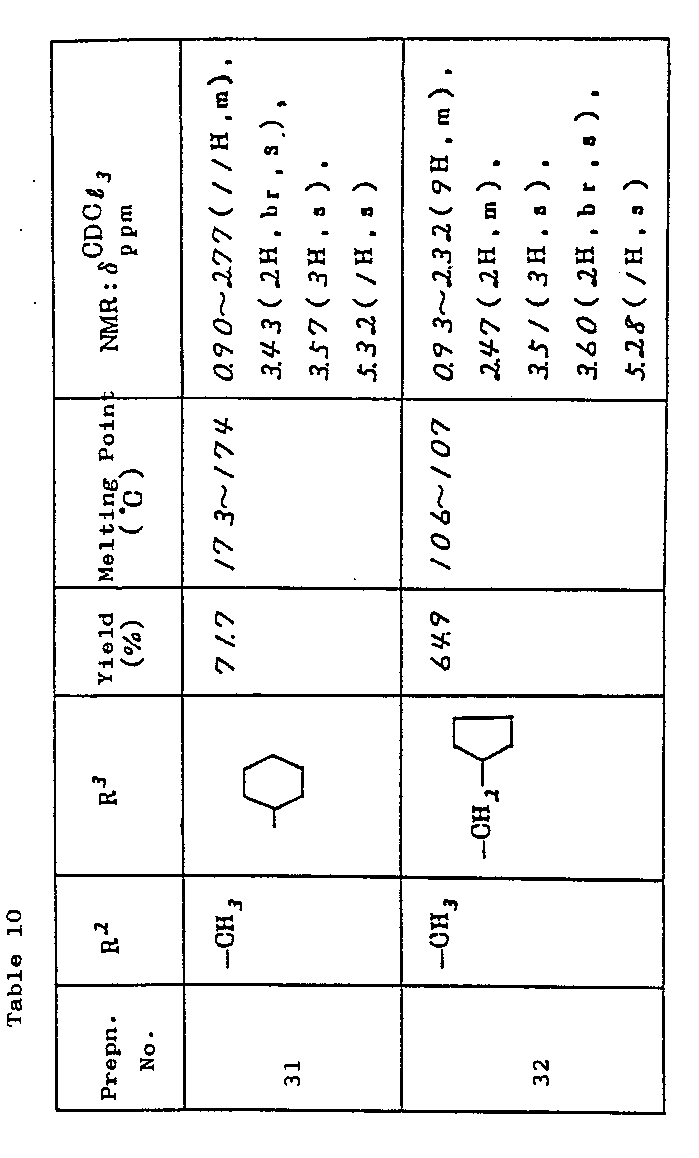

## Claims

1. 4,7-dihydropyrazolo[3,4-b]pyridine derivatives represented by the formula: 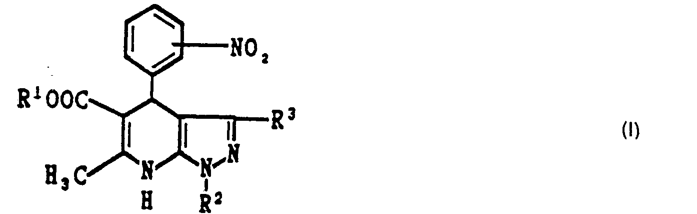 wherein R' is (1) C₇―C₈ alkyl, (2) C4―C₆ cycloalkyl which may be substituted by C₁―C₅ alkyls, (3) C₃-C₇ cycloalkyl(C₁―C₄)alkyl, (4) C₁―C₄ alkoxy(Cₗ-C₄)alkyl, (5) C₄-C₇ cycloalkyloxy(C₁―C₄)alkyl, (6) phenoxy(C₁―C₄)alkyl, (7) C₁―C₄ alkylthio(Cₗ-C₄)alkyl, (8) C4-C7 cycloalkylthio(C₁-C₄)alkyl, (9) phenylthio(C,-C₄)alkyl, (10) mono- or di-(C₁-C₄)alkylamino (Cₗ-C₄)alkyl, (11) tetrahydrofuryl-(C₁-C₄)alkyl, (12) phenyl(C₁-C₃)alkyl which may be substituted by one or two substituents selected from halogens and C₁-C₄ alkoxy, (13) N-benzylpyrrolidinyl, or (14) N-benzylpiperidinyl;
R² is C₁-C₄ alkyl; and R³ is C₄-C₆ cycloalkyl or C₃-C₇ cycloalkyl(C₁-C₄)alkyl; and acid-addition salts thereof.

2. A compound as claimed in claim 1, wherein the substituent-NO₂ is located at the 2 or 3 position of the phenyl group.

3. A compound as claimed in claim 1, wherein R² is methyl.

4. A compound as claimed in claim 1, wherein R³ is cyclopentyl or cyclohexyl.

5. A compound as claimed in claim 1, which is 2-isopropylthioethyl 3-cyclopentyl-1,6-dimethyl-4-(2-nitrophenyl)-4,7-dihydropyrazolo[3,4-b]pyridine-5-carboxylate.

6. A compound as claimed in claim 1, which is 2-cyclopentyloxyethyl 3-cyclopentyl-1,6-dimethyl-4-(2-nitrophenyl)-4,7-dihydropyrazolo[3,4-b]pyridine-5-carboxylate.

7. A compound as claimed in claim 1, which is 2-methylthioethyl 3-cyclopentyl-1,6-dimethyl-4-(2-nitrophenyl)-4,7-dihydropyrazolo[3,4-b]pyridine-5-carboxylate.

8. A compound as claimed in claim 1, which is 2-phenoxyethyl 3-cyclopentyl-1,6-dimethyl-4-(2-nitrophenyl)-4,7-dihydropyrazolo[3,4-b]pyridine-5-carboxylate.

9. A compound as claimed in claim 1, which is phehethyl 3-cyclopentyl-1,6-dimethyl-4-(2-nitrophenyl)-4,7-dihydropyrazolo[3,4-b]pyridine-5-carboxylate.

10. A compound as claimed in claim 1, which is 2-tetrahydrofurylmethyl 3-cyclopentyl-1,6-dimethyl-4-(2-nitrophenyl)-4,7-dihydropyrazolo[3,4-b]pyridine-5-carboxylate.

11. A compound as claimed in claim 1, which is 2-phenylthioethyl 3-cyclopentyl-1,6-dimethyl-4-(2-nitrophenyl)-4,7-dihydropyrazolo[3,4-b]pyridine-5-carboxylate.

12. A compound as claimed in claim 1, which is 2-cyclopentylthioethyl 3-cyclopentyl-1,6-dimethyl-4-(2-nitrophenyl)-4,7-dihydropyrazolo[3,4-b]pyridine-5-carboxylate.

13. A compound as claimed in claim 1, which is 2-cyclopentylethyl 3-cyclopentyl-1,6-dimethyl-4-(2-nitrophenyl)-4,7-dihydropyrazolo[3,4-b]pyridine-5-carboxylate.

14. A process for the production of 4,7-dihydropyrazolo[3,4-b]pyridine derivatives represented by the formula (I) 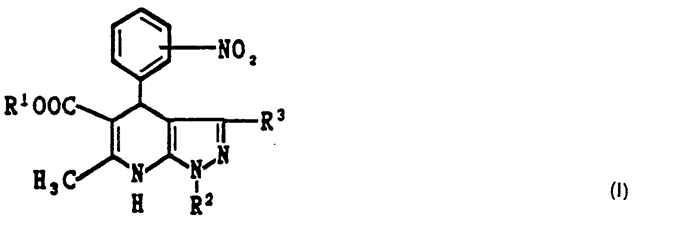 wherein R¹, R² and R³ have the same meanings as defined in claim 1, which comprises reacting a compound represented by the formula (II) 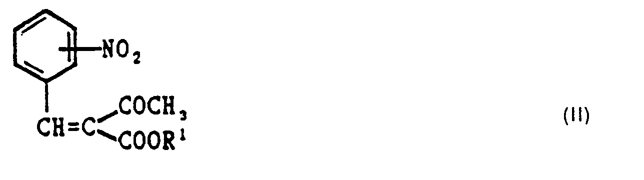 wherein R¹ has the same meaning as defined in claim 1, with a compound represented by the formula (III) 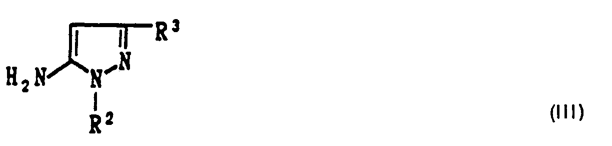 wherein R² and R³ have the same meanings as defined in claim 1.

15. Use of the derivatives of claim 1, for preparing a pharmaceutical composition against cardiovascular diseases.

## Patentansprüche

1. 4,7-Dihydropyrazolo[3,4-b]pyridinderivate der Formel: 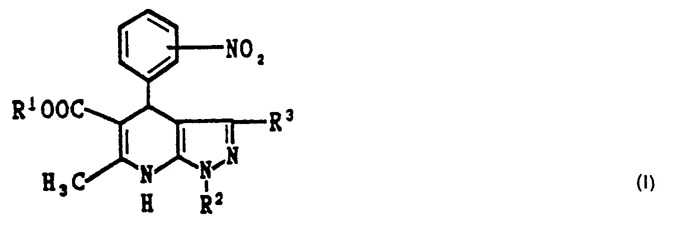 worin R' für (1) C₇-C₈-Alkyl, (2) C₄-C₆-Cycloalkyl, das durch C₁-C₅-Alkylgruppen substituiert sein kann, (3) C₃-C₇-Cycloalkyl-(C₁-C₄)-alkyl, (4) C₁-C₄-Alkoxy-(C₁-C₄)-alkyl, (5) C₄-C₇-Cycloalkyloxy-(C₁-C₄)-alkyl, (6) Phenoxy-(C₁-C₄)-alkyl, (7) C₁-C₄-Alkylthio-(C₁-C₄)-alkyl, (8) C₄-C₇-Cycloalkylthio-(C₁-C₄)-alkyl, (9) Phenylthio-(C₁-C₄)-alkyl, (10) Mono- oder Di-(C₁-C₄)-alkylamino-(C₁-C₄)-alkyl, (11) Tetrahydrofuryl-(C₁-C₄)-alkyl, (12) Phenyl-(C₁-C₃)-alkyl, das durch einen oder zwei Substituenten, ausgewählt aus Halogenatomen und C₁-C₄-Alkoxygruppen, substituiert sein kann, (13) N-Benzylpyrrolidinyl oder (14) N-Benzylpiperidinyl steht;
R² für C₁-C₄-Alkyl steht; und
R³ für C₄-C₆-Cycloalkyl oder C₃-C₇-Cycloalkyl-(C₁-C₄)-alkyl steht, und die Säureadditionssalze davon.

2. Verbindung nach Anspruch 1, dadurch gekennzeichnet, daß der Substituent -N0₂ in 2- oder 3-Position der Phenylgruppe angeordnet ist.

3. Verbindung nach Anspruch 1, dadurch gekennzeichnet, daß R² für Methyl steht.

4. Verbindung nach Anspruch 1, dadurch gekennzeichnet, daß R³ für Cyclopentyl oder Cyclohexyl steht.

5. Verbindung nach Anspruch 1, nämlich 2-Isopropylthioethyl-3-cyclopentyl-1,6-dimethyl-4-(2-nitrophenyl)-4,7-dihydropyrazolo[3,4-b]pyridin-5-carboxylat.

6. Verbindung nach Anspruch 1, nämlich 2-Cyclopentyloxyethyl-3-cyclopentyl-1,6-dimethyl-4-(2-nitrophenyl)-4,7-dihydropyrazolo[3,4-b]pyridin-5-carboxylat.

7. Verbindung nach Anspruch 1, nämlich 2-Methylthioethyl-3-cyclopentyl-1,6-dimethyl-4-(2-nitrophenyl)-4,7-dihydropyrazolo[3,4-b]pyridin-5-carboxylat.

8. Verbindung nach Anspruch 1, nämlich 2-Phenoxyethyl-3-cyclopentyl-1,6-dimethyl-4-(2-nitrophenyl)-4,7-dihydropyrazolo[3,4-b]pyridin-5-carboxylat.

9. Verbindung nach Anspruch 1, nämlich Phenethyl-3-cyclopentyl-1,6-dimethyl-4-(2-nitrophenyl)-4,7-dihydropyrazolo[3,4-b]pyridin-5-carboxylat. ,

10. Verbindung nach Anspruch 1, nämlich 2-Tetrahydrofurylmethyl-3-cyclopentyl-1,6-dimethyl-4-(2-nitrophenyl)-4,7-dihydropyrazolo[3,4-b]pyridin-5-carboxylat.

11. Verbindung nach Anspruch 1, nämlich 2-Phenylthioethyl-3-cyclopentyl-1,6-dimethyl-4-(2-nitrophenyl)-4,7-dihydropyrazolo[3,4-b]pyridin-5-carboxylat.

12. Verbindung nach Anspruch 1, nämlich 2-Cyclopentylthioethyl-3-cyclopentyl-1,6-dimethyl-4-(2-nitrophenyl)-4,7-dihydropyrazolo[3,4-b]pyridin-5-carboxylat.

13. Verbindung nach Anspruch 1, nämlich 2-Cyclopentylethyl-3-cyclopentyl-1,6-dimethyl-4-(2-nitrophenyl)-4,7-dihydropyrazolo[3,4-b]pyridin-5-carboxylat.

14. Verfahren zur Herstellung von 4,7-Dihydropyrazolo[3,4-b]pyridinderivaten der Formel (I): 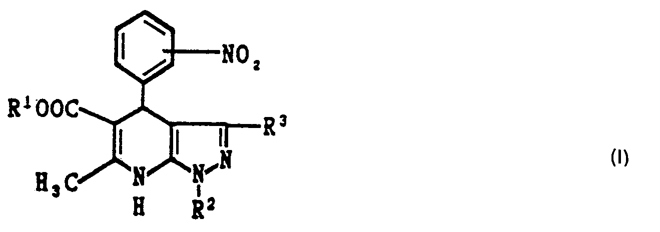 worin R¹, R² und R³ wie in Anspruch 1 definiert sind, dadurch gekennzeichnet, daß man eine Verbindung der Formel (II): 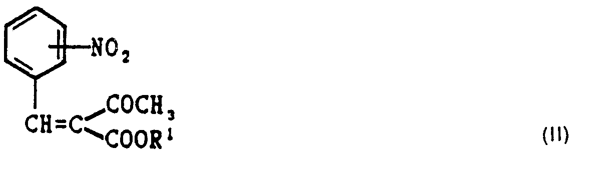 worin R¹ wie in Anspruch 1 definiert ist, mit einer Verbindung der Formel (III): 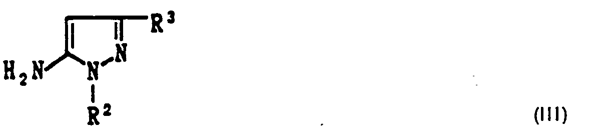 worin R² und R³ wie in Anspruch 1 definiert sind, umsetzt.

15. Verwendung der Derivate nach Anspruch 1 zur Herstellung eines pharmazeutischen Präparats gegen kardiovaskuläre Erkrankungen.

## Revendications

1. Dérivés de 4,7-dihydropyrazolo[3,4-b]pyridine, représentés par la formule: 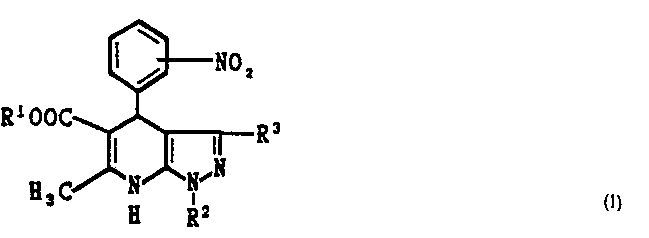 dans laquelle
R¹ est (1) un C₇-C₈ alkyle, (2) un C₄-C₆ cycloalkyle qui peut être substitué par des C₁-C₅ alkyles, (3) un C₃-C₇ cycloalkyl(C₁-C₄)alkyle, (4) un C₁-C₄ alkoxy(C₁-C₄)alkyle, (5) un C₄-C₇ cycloalkyloxy(C₁-C₄)alkyle, (6) un phénoxy(Cₗ-C₄)alkyle, (7) un C₁-C₄ alkylthio(C₁-C₄)alkyle, (8) un C₄-C₇ cycloalkylthio(C₁-C₄)alkyle, (9) un phénylthio(C₁-C₄)alkyle, (10) un mono- ou di-(C₁-C₄)alkylamino(C₁-C₄)alkyle, (11) un tétrahydrofuryl(C₁-C₄)alkyle, (12) un phényl(C₁-C₃)alkyle qui peut être substitué par un ou deux substituants choisis parmi les halogènes et C₁-C₄ alkoxy, (13) un N-benzylpyrrolidinyle ou (14) un N-benzylpipéridinyle;
R² est un C₁-C₄ alkyle; et
R³ est un C₄-C₆ cycloalkyle ou C₃-C₇ cycloalkyl(C₁-C₄)alkyle; et leurs sels d'addition d'acides.

2. Composé selon la revendication 1, caractérisé en ce que le substituant-N0₂ est situé en position 2 ou 3 dans le groupe phényle.

3. Composé selon la revendication 1, caractérisé en ce que R² est un méthyle.

4. Composé selon la revendication 1, caractérisé en ce que R³ est un cyclopentyle ou un cyclohexyle.

5. Composé selon la revendication 1, caractérisé en ce qu'il est un 2-isopropylthioéthyl 3-cyclopentyl-1,6-diméthyl-4-(2-nitrophényl)-4,7-dihydropyrazolo[3,4-b]pyridine-5-carboxylate.

6. Composé selon la revendication 1, caractérisé en ce qu'il est un 2-cyclopentyloxyéthyl 3-cyclopentyl-1,6-diméthyl-4-(2-nitrophényl)-4,7-dihydropyrazolo[3,4-b]pyridine-5-carboxylate.

7. Composé selon la revendication 1, caractérisé en ce qu'il est un 2-méthylthioéthyl 3-cyclopentyl-1,6-diméthyl-4-(2-nitrophényl)-4,7-dihydropyrazolo[3,4-b]pyridine-5-carboxylate.

8. Composé selon la revendication 1, caractérisé en ce qu'il est un 2-phénoxyéthyl 3-cyclopentyl-1,6-diméthyl-4-(2-nitrophényl)-4,7-dihydropyrazolo[3,4-b]pyridine-5-carboxylate.

9. Composé selon la revendication 1, caractérisé en ce qu'il est un phényléthyl 3-cyclopentyl-1,6-diméthyl-4-(2-nitrophényl)-4,7-dihydropyrazolo[3,4-b]pyridine-5-carboxylate.

10. Composé selon la revendication 1, caractérisé en ce qu'il est un 2-tétrahydrofurylméthyl 3-cyclopentyl-1,6-diméthyl-4-(2-nitrophényl)-4,7-dihydropyrazolo[3,4-b]pyridine-5-carboxylate.

11. Composé selon la revendication 1, caractérisé en ce qu'il est un 2-phénylthioéthyl 3-cyclopentyl-1,6-diméthyl-4-(2-nitrophényl)-4,7-dihydropyrazolo[3,4-b]pyridine-5-carboxylate.

12. Composé selon la revendication 1, caractérisé en ce qu'il est un 2-cyclopentylthioéthyl 3-cyclopentyl-1,6-diméthyl-4-(2-nitrophényl)-4,7-dihydropyrazolo[3,4-b]pyridine-5-carboxylate.

13. Composé selon la revendication 1, caractérisé en ce qu'il est un 2-cyclopentyléthyl 3-cyclopentyl-1,6-diméthyl-4-(2-nitrophényl)-4,7-dihydropyrazolo[3,4-b]pyridine-5-carboxylate.

14. Procédé de préparation de dérivés de 4,7-dihydropyrazolo[3,4-b]pyridine représentés par la formule (I): 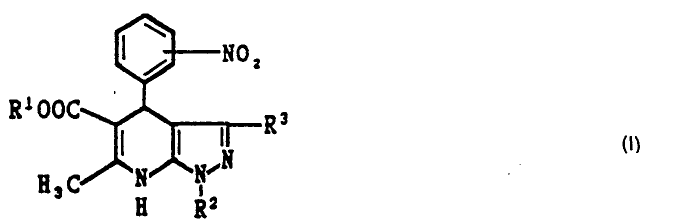 dans laquelle R', R² et R³ ont la même signification que dans la revendication 1, caractérisé en ce qu'on fait réagir un composé représenté par la formule (II): 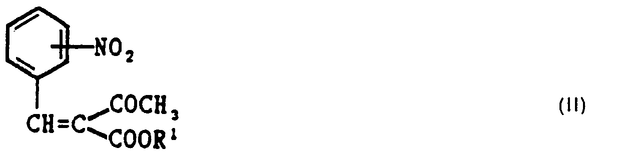 dans laquelle R' a la même signification que dans la revendication 1, avec un composé représenté par la formule (III): 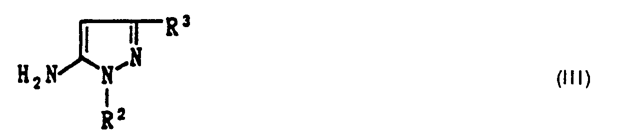 dans laquelle R² et R³ ont la même signification que dans la revendication 1.

15. Utilisation des dérivés selon la revendication 1 pour préparer une composition pharmaceutique pour le traitement des maladies cardio-vasculaires.
